(19) Europäisches Patentamt / European Patent Office / Office européen des brevets

(11) **EP 4 308 682 B1**

(12) **EUROPÄISCHE PATENTSCHRIFT**

(45) Veröffentlichungstag und Bekanntmachung des Hinweises auf die Patenterteilung:
**12.03.2025 Patentblatt 2025/11**

(21) Anmeldenummer: **22714183.5**

(22) Anmeldetag: **14.03.2022**

(51) Internationale Patentklassifikation (IPC):
**C12M 3/00** *(2006.01)* **C12M 1/00** *(2006.01)*
**C12M 1/34** *(2006.01)* **C12M 1/36** *(2006.01)*

(52) Gemeinsame Patentklassifikation (CPC):
**C12M 41/48; C12M 23/44; C12M 23/50; C12M 41/36**

(86) Internationale Anmeldenummer:
**PCT/EP2022/056442**

(87) Internationale Veröffentlichungsnummer:
**WO 2022/194730 (22.09.2022 Gazette 2022/38)**

(54) **SYSTEM ZUM KULTIVIEREN BIOLOGISCHER ZELLKULTUREN**

SYSTEM FOR CULTIVATING BIOLOGICAL CELL CULTURES

SYSTÈME DE MISE EN CULTURE DE CULTURES CELLULAIRES BIOLOGIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.03.2021 DE 102021106852**
**14.12.2021 DE 102021133108**

(43) Veröffentlichungstag der Anmeldung:
**24.01.2024 Patentblatt 2024/04**

(73) Patentinhaber: **Aixinno Limited**
**Cambridge, Cambridgeshire CB24 4FQ (GB)**

(72) Erfinder:
• **GILLIGAN, Patrick Clemente**
**Cambridge Cambridgeshire CB1 3SU (GB)**
• **TEO, Kenneth B. K.**
**Cambridge Cambridgeshire CB23 5BH (GB)**
• **CONNAH, John Edward**
**Cambridge Cambridgeshire CB 25 OEJ (GB)**
• **BULLINARIA, David Anthony**
**Bluntisham, Huntingdon PE28 3XD (GB)**

(74) Vertreter: **Grundmann, Dirk et al**
**Rieder & Partner mbB**
**Patentanwälte - Rechtsanwalt**
**Yale-Allee 26**
**42329 Wuppertal (DE)**

(56) Entgegenhaltungen:
EP-A1- 1 598 415    WO-A1-2019/241885
WO-A1-2020/098957    WO-A1-2020/098960
US-A1- 2005 260 743    US-A1- 2019 033 819
US-A1- 2020 283 713

**Beschreibung**

Gebiet der Technik

Die Erfindung betrifft ein System oder ein Verfahren zum Kultivieren biologischer Zellkulturen, mit einer Vielzahl von Trägern, die jeweils ein oder mehrere Zellkulturen tragen, mit mindestens einem Aufbewahrungsmodul, in dem die Träger aufbewahrt werden, mit einem Prozessmodul, in dem die von den Trägern getragenen Zellkulturen mittels zumindest einer Behandlungseinrichtung behandelt werden, mit einer Transporteinrichtung, mit der die Träger zwischen dem Aufbewahrungsmodul und dem Prozessmodul transportiert werden und mit einer programmierten oder programmierbaren Steuereinrichtung, die eine Speichereinrichtung aufweist und die die Transporteinrichtung und die Behandlungseinrichtung steuert, wobei in der Speichereinrichtung mehreren voneinander verschiedenen Zellkulturen zugeordnete Applikationen gespeichert sind, wobei jede Applikation eine Behandlungsvorschrift zur Behandlung der zugeordneten Zellkultur ist, die ein oder mehrere Behandlungseinheiten, nachfolgend mit Workflows bezeichnet, beinhaltet, die in zeitlichen Abständen nacheinander jeweils zu einer Startzeit in dem Prozessmodul durchgeführt werden, wobei ein Workflow ein oder mehrere Arbeitsschritte beinhaltet, wobei die ein oder mehreren Arbeitsschritte unmittelbar aufeinanderfolgend durchgeführt werden müssen, wobei die Steuereinrichtung die gleichzeitige Durchführung einer Mehrzahl von Applikationen steuert und dabei die ein oder mehreren Workflows der voneinander verschiedenen Applikationen in einer zeitlichen Reihenfolge hintereinander im selben Prozessmodul startet.

**Stand der Technik**

Zellen, insbesondere menschliche Zellen, werden in Kunststoffschalen, beispielsweise in Mulden von Trägern, durch Zugabe von Nährstoffen gezüchtet. Adhärente Zellen werden typischerweise in einer Monolage gezüchtet, die am Boden einer Kunststoffschale oder einer Mulde in einem Träger anhaftet. Derartige Zellen werden vom Boden abgelöst, bevor der gesamte Boden mit den Zellen bedeckt ist. Hierzu wird ein Konfluenz-Scan durchgeführt, mit dem der Bedeckungsgrad eines Substrates, beispielsweise des Bodens eines Trägers mit Zellen ermittelt wird. Die vom Boden abgelösten Zellen können verdünnt und auf neue Träger beziehungsweise in neue Mulden übertragen werden. Systeme und Verfahren des Standes der Technik zur Pflege derartiger Zellkulturen können einen Inkubator besitzen, einen Roboter für das Handhaben der die Zellen tragenden Träger und Behandlungseinrichtungen, mit denen das Nährmedium zugefügt beziehungsweise gewechselt werden kann.

US 2005/260743 beschreibt ein modulares Zellkultursystem und Verfahren mit unterschiedlichen Behandlungsstationen, sog. Manipulationsmodulen, unter Verwendung eines Workflow-Managements, welches auch 'automated, smart (intelligent) system' genannt wird. Es werden im Computer und den Programmen 'operational rules' und 'process rules' in die Software geschrieben und hinterlegt.

Die EP 1 598 415 A1 beschreibt ein System zum Kultivieren biologischer Zellkulturen, bei dem Zellen auf Trägern in einem Aufbewahrungsmodul bevorratet werden. Mit einer Transporteinrichtung, die einen Greifer umfasst, können die Träger aus dem Aufbewahrungsmodul entnommen werden und in ein Prozessmodul transportiert werden. Dort werden die auf dem Träger bevorrateten Zellkulturen mittels einer Behandlungseinrichtung behandelt. Verschiedenartige Zellkulturen werden nach voneinander verschiedenen Behandlungsvorschriften behandelt. Zu jeder Zellkultur, beispielsweise zu jedem Träger, kann es eine Applikation geben, die eine Behandlungsvorschrift zur Behandlung der zugeordneten Zellkultur ist. Eine derartige Applikation kann ein oder mehrere Workflows beinhalten. Jeder Workflow kann zumindest einen Arbeitsschritt beinhalten. Ein Workflow kann aber auch mehrere Arbeitsschritte aufweisen. Die Arbeitsschritte eines Workflows zeichnen sich dadurch aus, dass sie unmittelbar oder kurzfristig aufeinanderfolgend durchgeführt werden müssen. Die mehreren Workflows eine Applikation werden demgegenüber jeweils in größeren zeitlichen Abständen zwischen einzelnen Workflows durchgeführt. Zwischen der Durchführung einzelner Workflows können sich die Träger in ihnen zugeordneten Lagerplätzen im Aufbewahrungsmodul befinden. Die Applikationen, deren Anzahl 100 und mehr betragen kann, und die ihnen zugeordneten Workflows können in einer Speichereinrichtung einer Steuereinrichtung als digitale Datensätze abgespeichert sein. Die programmierbare Steuereinrichtung koordiniert die parallele Durchführung einer Mehrzahl von Applikationen und steuert die ein oder mehreren Workflows der voneinander verschiedenen Applikationen in einer zeitlichen Reihenfolge hintereinander im selben Prozessmodul.

Ein automatisiertes System für die Durchführung dieser Applikationen, die bevorzugt zu mindestens ein oder mehreren Workflows beinhalten, in denen das Nährmedium gewechselt wird beziehungsweise eine Konfluenz gemessen wird, werden von einem Planungsprogramm der Steuereinrichtung gesteuert. Ein derartiges Planungsprogramm erstellt einen Zeitplan, um die Workflows der einzelnen Applikationen hintereinander durchzuführen. Das Planungsprogramm sollte darüber hinaus in der Lage sein, die Workflows zu beeinflussen. Im Allgemeinen enthält ein derartiger Zeitplan mehrere Arbeitsabläufe, bei denen die Dauer der Arbeitsabläufe und die Intervalle zwischen den Arbeitsabläufen auf unvorhersehbare Weise variieren können. Da Zellen lebende Einheiten sind und ihre Eigenschaften, insbesondere Wachstumsraten, auf unvorhersehbare Weise variieren können, müssen die Arbeitsabläufe an die jeweilige individuelle Entwicklung der Zellkultur angepasst werden. Im Allgemeinen ist die Wachstumsrate von Zellen variabel und ist schwer vorhersehbar. Die Wachstumsrate der Zellen kann beispielsweise von Charge zu Charge aufgrund bekannter Faktoren, wie Transfection, Serummangel oder kryptischer Faktoren variieren. Das Verhalten insbesondere der Wachstumsrate einer Zellkultur muss somit beobachtet werden, um Zeitabläufe hinsichtlich der Zeitpunkte des Starts eines Workflows dem Entwicklungsstand einer Zellkultur anzupassen.

Ein System zum Kultivieren biologischer Zellkulturen muss darüber hinaus in der Lage sein, die Applikationen zeiteffektiv durchzuführen und die Startzeiten der Workflows derart festlegen, dass Workflows, die zu einer optimalen Startzeit in die Behandlungsdauer eines anderen Workflows fallen würden, zeitlich derart verschoben werden, dass sie nacheinander in dem Prozessmodul durchgeführt werden.

Zusammenfassung der Erfindung

Der Erfindung liegt die Aufgabe zugrunde, ein System oder ein Verfahren zum Kultivieren biologischer Zellkulturen, bei dem Applikationen mit Workflows zeitgleich durchgeführt werden, gebrauchsvorteilhaft weiterzubilden. Eine Aufgabe besteht insbesondere darin, das System kostengünstig zu betreiben.

Gelöst wird die Aufgabe durch die in den Ansprüchen angegebene Erfindung, wobei die Unteransprüche nicht nur vorteilhafte Weiterbildungen der im Anspruch 1 angegebenen Erfindung sind, sondern auch eigenständige Lösungen der Aufgabe darstellen.

Ein erster Aspekt der Erfindung betrifft das Vermeiden zeitlicher Kollisionen von Workflows, die unter optimalen Bedingungen sich überlappende Bearbeitungszeiten hätten. Derartige Workflows müssen zeitlich derart verschoben werden, dass die Startzeit des einen Workflows nach der Beendigung des anderen Workflows liegt. Erfindungsgemäß wird vorgeschlagen, dass jedem Workflow eine von der Startzeit abhängige Wirkfunktion zugeordnet ist. Die Wirkfunktion beziehungsweise die Kurve der Wirkfunktion kann ein Extremum zu der Zeit aufweisen, bei der der Workflow bevorzugt durchgeführt werden würde. Die Wirkfunktion ist somit minimal oder maximal beziehungsweise die Kurve der Wirkfunktion hat ein Minimum oder Maximum zu einer Zeit, bei der der Workflow bevorzugt durchgeführt werden würde. Bevorzugt ist das Minimum der Wirkfunktion. Die Wirkfunktion steigt dann zu früheren Zeiten und zu späteren Zeiten an. Der Anstieg kann linear oder nicht linear sein und in Richtung einer frühesten Startzeit schwächer ansteigen, als in Richtung einer spätesten Startzeit oder anders herum. Handelt es sich bei dem Workflow beispielsweise um einen Workflow, bei dem der Zustand einer Zellkultur überwacht werden soll, so kann der Workflow einen Arbeitsschritt aufweisen, in dem der Bedeckungsgrad des Bodens einer Mulde, in der die Zellen bevorratet werden, mit den Zellen gemessen wird. Ein derartiger Konfluenz-Scan kann mithilfe eines Mikroskops und einer automatischen Bildauswertung durchgeführt werden. Abhängig vom Bedeckungsgrad kann in einem Folgeschritt eine Zellentnahme stattfinden, um den Bedeckungsgrad zu vermindern. Es können Flüssigkeiten zugegeben werden, um die Zellen vom Boden abzulösen. Die Entnahme der Zellen kann mit einer Pipettiereinrichtung erfolgen. Gleichzeitig kann durch die Messung des Bedeckungsgrades eine der Zellkultur individuell zugeordnete Wachstumsrate bestimmt werden, in dem ein Bedeckungsgrad einer früheren Messung unter Berücksichtigung der in der Zwischenzeit verstrichenen Zeit mit dem aktuellen Bedeckungsgrad verglichen wird. Mit dieser durch den Konfluenz-Scan gemessenen Wachstumsrate kann ein zukünftiger Zeitpunkt berechnet werden, wann die nächste Überwachung der Zellkultur in einem folgenden Workflow durchzuführen ist. Das System berechnet eine bevorzugte Startzeit. Die bevorzugte Startzeit kann beispielsweise eine Zeit sein, bei der aufgrund der ermittelten Wachstumsrate voraussichtlich eine Konfluenz von 80% gegeben sein wird. Das System kann eine späteste Startzeit berechnen, die etwa dort liegt, wo aufgrund der ermittelten Wachstumsrate eine Konfluenz von 90% bis 100% vorhergesagt wird. Eine spätere Startzeit würde ein Überwachsen der Oberfläche verursachen. Das System kann eine früheste Startzeit berechnen, die beispielsweise dort liegt, wo die berechnete Konfluenz zwischen 15% und 30%, bevorzugt 20% liegt. Mit diesen Randwerten der frühesten Startzeit, der spätesten Startzeit und der bevorzugten Startzeit wird eine Wirkfunktion konstruiert, die normiert sein kann, beispielsweise zur bevorzugten Startzeit den Wert 0 und zur frühesten Startzeit beziehungsweise spätesten Startzeit den Wert 1 aufweist. Es kann sich um eine Funktion der bevorzugten Startzeit handeln. Es ist aber auch möglich, dass eine derartige Wirkfunktion insbesondere zur spätesten Startzeit asymptotisch langsam oder schnell zum Maximalwert ansteigt, wenn die bevorzugte Startzeit sich dem frühesten oder spätesten zulässigen Wert nähert. Enthält das System Applikationen, die jeweils zumindest einen Workflow aufweisen, der aufgrund seiner bevorzugten Startzeit in die Behandlungsdauer eines anderen Workflows fallen würde, so werden in einer Variationsrechnung die Startzeiten der beiden Workflows derart variiert, dass die Summe der beiden Wirkfunktionen minimiert ist beziehungsweise ein Minimum aufweist. Beinhaltet das System Applikationen, bei denen mehrere Work-lows sich hinsichtlich ihrer Durchführungszeit überlappen werden, so werden diese Workflows einer Variationsrechnung unterzogen, bei der die Startzeiten der Workflows derart variiert werden, dass die Summe aller Wirkfunktionen ein Minimum aufweist. Bei dem Minimum kann es sich um ein lokales Minimum handeln. Es kann sich aber auch um ein globales Minimum handeln. Es ist insbesondere vorgesehen, dass zumindest einigen der Workflows eine früheste Startzeit zugeordnet ist, vor der der Workflow nicht gestartet werden darf. Es ist aber auch vorgesehen, dass zumindest einige der Workflows eine späteste Startzeit aufweisen, nach der der Workflow nicht gestartet werden darf. Ferner kann vorgesehen sein, dass die früheste Startzeit und die späteste Startzeit unmittelbar neben der bevorzugten Startzeit liegen, der Workflow somit nur zur bevorzugten Startzeit gestartet werden darf. Eine diesbezügliche Wirkfunktion kann mathematisch dadurch dargestellt werden, dass sie ein oder zwei sehr steile Äste aufweist, deren Steigung schnell ansteigt, wenn die Funktion sich dem frühesten oder spätesten Startwert annähert. Im einfachsten Fall können die Wirkfunktionen linear verlaufende Äste aufweisen. Die Äste der Wirkfunktion können aber auch durch ein Polynom auch höheren Grades dargestellt werden. Gemäß einer bevorzugten Ausführungsform der Erfindung werden die tatsächlichen Startzeiten der Wirkfunktionen für die Workflows so gewählt, dass die Summe der Wirkfunktionen für die Workflows minimiert wird, da die Wirkfunktion erhöht wird, wenn ein Workflow zu einer anderen Startzeit als der bevorzugten Startzeit ausgeführt wird. Auf diese Weise kann die Wirkfunktion als Kostenfunktion betrachtet werden, wobei die Ausführung eines Workflows zu früh oder zu spät eine Strafe oder Kosten verursacht. Ziel des Systems ist es, die Summe der Wirkfunktionen oder Kostenfunktionen über die Workflows der Applikationen zu minimieren, d.h. die Zeiteffizienz des Systems zu maximieren.

Die Erfindung betrifft u.a. auch eine Planungssoftware oder einen Planer zum Verwalten mehrerer Funktionen mit ein oder mehreren Workflows. In einer alternativen Ausgestaltung eines derartigen Planers können die Funktionen und das Ziel des Planers auch umgekehrt werden. Eine Funktion könnte zu einem Zeitpunkt maximal werden, zu dem ein Arbeitsablauf gestartet wird. Das Ziel des Systems wäre es, die Summe der Wirkfunktionen über die Workflows der Applikationen zu maximieren, um die Zeiteffizienz des Systems zu maximieren.

Ein Workflow kann zumindest ein oder mehrere der folgenden Arbeitsschritte umfassen: Anlegen einer Zellkultur, Zählen der lebenden oder toten Zellen, Lösen der Zellen vom Boden einer Mulde eines Trägers, Entnahme von Zellen der Zellkultur zur Verminderung des Bedeckungsgrades beziehungsweise der Konfluenz, Konfluenz-Scan, Wechseln eines Mediums der Zellkultur, Nährstoffzugabe oder Aufgabe der Zellkultur. Es kann insbesondere vorgesehen sein, dass die Workflows in einer Applikation zeitlich weit verteilt sind, in dem Sinne, dass die Workflows große zeitliche Abstände untereinander besitzen. Ein Workflow kann beispielsweise 30 Minuten dauern. Während dieser Zeit kann ein Konfluenz-Scan durchgeführt werden, das Medium der Zellkultur gewechselt werden oder eine Nährstoffzugabe stattfinden. Die Zeit zwischen zwei aufeinanderfolgenden Workflows kann einige Stunden oder sogar mehrere Tage betragen, wobei die Zwischenzeit zwischen aufeinanderfolgenden Workflows von einer stetig oder jeweils bei der Durchführung eines Workflows neu berechneten Wachstumsrate der Zellkultur abhängt. So kann vorgesehen sein, dass bei einem Workflow, bei dem das Zellwachstum überwacht wird, zunächst ein Konfluenz-Scan durchgeführt wird, mit dem auf Basis der beim vorherigen Workflow eingestellten Konfluenz die Wachstumsrate bestimmt wird. Anschließend kann die Konfluenz durch Entnahme von Zellen auf beispielsweise 10% reduziert werden. Anschließend kann die Konfluenz auf beispielsweise 10% reduziert werden, indem Zellen entfernt, abgetrennt, verdünnt oder in einem neuen Träger ausgesät werden. Auf Basis der ermittelten Wachstumsrate kann die bevorzugte Startzeit ($t_p$) des folgenden Workflows derart bestimmt werden, dass die voraussichtliche Konfluenz 80% beträgt. Auf Basis der ermittelten Wachstumsrate wird auch die Wirkfunktion neu ermittelt, beispielsweise durch Festlegen einer frühesten Startzeit, bei der die Konfluenz 20% beträgt und einer spätesten Startzeit ($t_l$), bei der die voraussichtliche Konfluenz 95% beträgt.

Die Arbeitsschritte innerhalb eines Workflows bilden zeitlich zusammenhängende Abläufe, die nacheinander, zum Beispiel mit zeitlichen Abständen von idealerweise 0 (Null) oder höchstens wenigen Minuten durchgeführt werden müssen, wobei zwischen den Arbeitsschritten kein anderer Workflow eingefügt werden kann. Ein erster Arbeitsschritt kann beispielsweise die Entnahme eines Trägers aus dem Aufbewahrungsmodul und sein Transport zum Prozessmodul sein. Ein darauf folgender zweiter Arbeitsschritt kann beispielsweise ein Konfluenz-Scan sein, bei dem der Bedeckungsgrad der am Boden einer Schale anhaftenden Zellen mit optischen Mitteln ermittelt wird. Ein dritter Arbeitsschritt kann die Zugabe eines "Detachmentagent" sein, mit dem die Zellen vom Boden der Schale oder Mulde des Trägers abgelöst werden. Ein vierter Arbeitsschritt kann die zumindest teilweise Entnahme der Zellen aus der Nährlösung in der Schale oder Mulde sein oder die Überführung eines Teils der Zellen in eine frische Schale oder Mulde. Mit diesem Schritt kann der Bedeckungsgrad, also die Konfluenz, eingestellt werden. Ein fünfter Arbeitsschritt kann die Zugabe von Nährlösung sein. Ein sechster Arbeitsschritt kann der Transport des Trägers zurück zum Aufbewahrungsmodul sein. Jeder dieser Arbeitsschritte kann den Verlauf der Wirkfunktion beeinflussen. Weitere Arbeitsschritte können das Zählen der lebenden und der toten Zellen sein. Dieser Arbeitsschritt kann vor oder nach dem zweiten Arbeitsschritt durchgeführt werden. Für die Kultivierung einer bestimmten Zelllinie, z.B. CHO-Zellen (Chinese Hamster Ovary), wird eine Instanz einer Applikation erstellt, die die Parameter für die Kultivierung dieser Zelllinie enthält, beispielsweise ein komplettes Medium-Rezept, die Konfluenz, bei der die Zellen zu passieren sind, ein Split-Verhältnis und die Anzahl der Trägerplatten (dishes). Eine Applikation kann ein oder mehrere Trägerplatten betreffen. Bei einer Applikation, die mehrere Trägerplatten betrifft, die jeweils wiederum eine Vielzahl von Schalen, Mulden oder Träger aufweist, in denen Zellkulturen bevorratet werden, besitzt Workflows, deren zeitliche Länge größer ist, als bei Applikationen, die nur eine Trägerplatte betreffen. Das Intervall zwischen zwei Workflows hängt im Wesentlichen von der Zelllinie und hauptsächlich von der Verdopplungszeit beziehungsweise dem Splitverhältnis der Zellen der Zelllinie ab. Das Split-Verhältnis gibt den Verdünnungsgrad der Zellen bei jedem Workflow an. Üblicherweise variiert die Verdopplungszeit von Zelllinie zu Zelllinie und damit auch von Instanz zu Instanz einer Applikation zur Zelllinienpflege. Es gibt zeitkritische Workflows, die durchgeführt werden müssen, um zu vermeiden, dass die Zellen überkonfluent werden.

Eine Applikation kann Workflows enthalten, die in Anzahl und Reihenfolge variieren. Zum Beispiel enthält die Wartung von iPSCs (induzierte Pluripotente Stammzellen) typischerweise einen Workflow für den Medienwechsel ("Füttern von Zellen" oder "erneutes Füttern von Zellen"), da iPSCs Faktoren wie FGF2 eine kurze Halbwertzeit benötigen. Stammzellen müssen typischerweise alle 24 Stunden gefüttert werden. Die Frequenz eines "Mediumwechsels" kann variieren und ist abhängig von der Wachstumsrate beziehungsweise dem Teilungsverhältnis der Zellen. Die Zeitpunkte der Konfluenz-Scans können ebenfalls von der Wachstumsrate der Zellen abhängen, sodass ein Konfluenz-Scan z.B. vor oder nach einem ersten oder zweiten Medienwechsel erfolgen kann.

Die obigen Ausführungen charakterisieren die Wirkfunktion als eine Funktion, die zu minimieren ist, wenn beispielsweise die Wirkfunktion Kosten repräsentiert. Der umgekehrte Fall ist aber auch möglich, wenn beispielsweise die Wirkfunktion einen Nutzen repräsentiert. Dann ist die Wirkfunktion jeweils zu maximieren.

<u>Kurze Beschreibung der Zeichnungen</u>

Ausführungsbeispiele der Erfindung werden nachfolgend anhand beigefügter Zeichnungen erläutert. Es zeigen:

Fig. 1 in dem mit a bezeichneten oberen Abschnitt drei Applikationen A1, A2, A3 mit jeweils zugehörigen Workflows W1.1, W1.2, W1.3; W2.1, W2.2, W2.3 und W3.1, W3.2 und W3.3, die zu bevorzugten Startzeiten durchzuführen sind, wobei die Workflows W2.1, W2.2 und W2. 3 der Applikation A2 zu festen (unveränderbar festgelegten) Startzeiten durchgeführt werden müssen,
in dem mit b bezeichneten unteren Abschnitt die Workflows der drei Applikationen, nachdem sie teilweise zeitlich verschoben (siehe Pfeil) derart angeordnet worden sind, dass sie in einem Prozessmodul hintereinander durchgeführt werden können,

Fig. 2 in dem mit a bezeichneten oberen Abschnitt eine Wirkfunktion W(t),

mit dem mit b bezeichneten darunterliegenden Abschnitt eine Zeitskala in Tagen und

mit dem mit c bezeichneten unteren Abschnitt die zur Zeitskala korrespondierenden Werte der Konfluenz,

Fig. 3 ein Berechnungsschema zum Berechnen der frühesten Startzeit ($t_e$), der bevorzugten Startzeit ($t_p$) und der spätesten Startzeit ($t_l$),

Fig. 4 ein Berechnungsschema ebenfalls zum Berechnen der bevorzugten Startzeit aus dem Ergebnis eines Konfluenz-Scans,

Fig. 5 eine Darstellung gemäß Figur 2a einer Wirkfunktion W(t),

Fig. 6 eine weitere Darstellung gemäß Figur 2a einer Wirkfunktion W(t),

Fig. 7 eine Applikation A, die drei Workflows W1, W2, W3 beinhaltet, die zyklisch wiederholt werden,

Fig. 8 eine Applikation A, die fünf Workflows W1, W2, W3, W4, W5 beinhaltet, die zyklisch wiederholt werden,

Fig. 9 schematisch die Schritte zur Lösung eines zeitlichen Konflikts, wenn zwei oder mehr Workflows zeitgleich durchzuführen sind beziehungsweise die Startzeiten eines Workflows in die Durchführungszeit eines anderen Workflows fällt;

Fig. 10 schematisch die Elemente eines erfindungsgemäßen Systems,

Fig. 11 schematisch eine grafische Oberfläche (user interface) zur manuellen Gestaltung einer Wirkfunktion,

Fig. 12 eine Darstellung gemäß Figur 1, bei der zur Optimierung der Wirkfunktion, beispielsweise Maximieren oder Minimieren zwei Workflows W2.1 und W3.2 in ihrer zeitlichen Reihenfolge getauscht worden sind,

Fig. 13 eine Darstellung gemäß Figur 2, jedoch für den Fall, dass die Wirkfunktion einen Nutzen angibt und zu maximieren ist.

## Beschreibung der Ausführungsformen

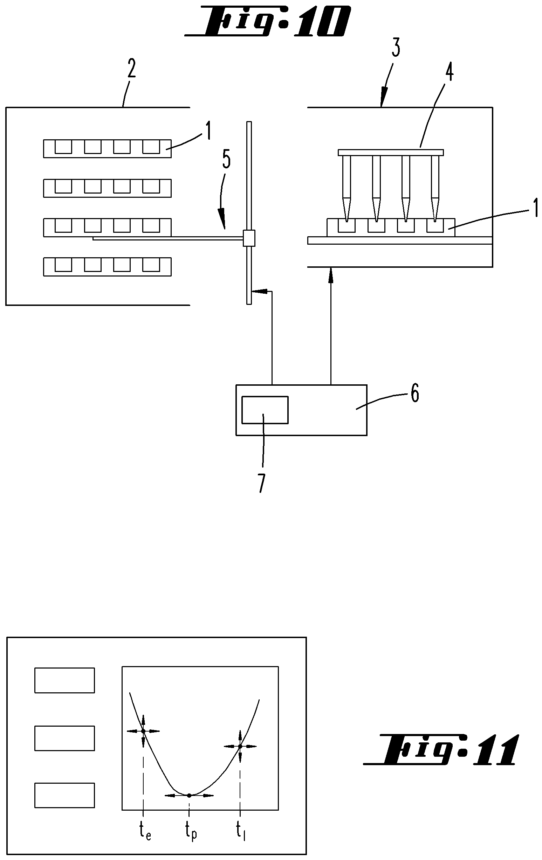

Die Figur 10 zeigt schematisch ein System bestehend aus einem Aufbewahrungsmodul 2 zum Aufbewahren von Zellkulturen. Derartige Aufbewahrungsmodule 2 können Inkubatoren sein. Im Gehäuse eines derartigen Aufbewahrungsmoduls können sich mehrere "Hotels" befinden, in denen übereinander eine Vielzahl von Trägern 1 bevorratet werden. Hinsichtlich derartiger Aufbewahrungsmodule 2 wird auf die WO 2020/098960 A1 verwiesen.

Es ist eine Transporteinrichtung 5, die einen Greifer, Endeffektor und/oder anderweitige Transportmittel, beispielsweise Transportschienen oder einen Automatisierungsmechanismus aufweisen können, vorgesehen, mit der ein Träger 1 vom Aufbewahrungsmodul 2 zu einem Prozessmodul 3 transportiert werden kann. Hinsichtlich der Ausgestaltung eines Prozessmoduls wird auf die WO 2020/098957 A1 verwiesen. In dem Prozessmodul 3 befinden sich ein oder mehrere Behandlungseinrichtungen 4. Die Behandlungseinrichtung 4 kann ein Mikroskop, eine Pipettiereinrichtung oder dergleichen aufweisen.

Die bevorzugt mehreren Träger 1, die in dem Aufbewahrungsmodul 2 bevorratet werden, können Mikroplatten sein. Derartige Mikroplatten besitzen eine Vielzahl von Mulden, in denen jeweils ein Nährmittel mit Zellkulturen bevorratet wird. Jeder Träger 1 kann Zellkulturen eines Zelltypen aufweisen. Verschiedene Träger 1 können verschiedene Zelllinien tragen. Das Aufbewahrungsmodul 2 ist so ausgebildet, dass die dort bevorrateten Zellen sich mit einer zellspezifischen Teilungsrate vermehren.

Eine Steuereinrichtung 6 kann einen Mikrocontroller oder einen Mikrocomputer aufweisen, der in der Lage ist, die Transporteinrichtung 5 und die Behandlungseinrichtung 4 zu steuern. Die Steuereinrichtung 6 kann darüber hinaus eine Speichereinrichtung 7 aufweisen, in der in digitaler Form Applikationen gespeichert sind. Eine Applikation ist eine Behandlungsvorschrift zur Behandlung von Zellkulturen, wobei vorgesehen sein kann, dass jedem Träger 1 eine Applikation, also eine Behandlungsvorschrift, zugeordnet ist, die die Eigenarten der vom Träger 1 getragenen Zelllinie berücksichtigt. Eine Applikation A kann aber auch mehrere Träger 1 umfassen, wobei dann insbesondere vorgesehen ist, dass die mehreren Träger 1 Zellen desselben Typs tragen. In dem Aufbewahrungsmodul 2 können Träger 1 bevorratet werden, die jeweils voneinander verschiedene Zellkulturlinien tragen und denen jeweils eine andere Applikation A1, A2, A3 zugeordnet ist. Jede Applikation kann ein oder mehrere Workflows W1, W2, W3, W4, W5 aufweisen, wobei jeder Workflow ein oder mehrere Arbeitsschritte beinhalten kann. Die Workflows einer Applikation A1, A2, A3 sind in vorgegebenen Zeitintervallen nacheinander durchzuführen, wobei jeder Workflow W1, W2, W3 eine bevorzugte Startzeit aufweist, in der er gestartet werden kann. Einige oder alle Workflows W1, W2, W3 können aber auch zu einem früheren Zeitpunkt oder zu einem späteren Zeitpunkt gestartet werden, jedoch nicht vor einer frühesten Startzeit $t_e$ und nicht nach einer spätesten Startzeit $t_l$. Zwischen den Workflows einer Applikation können Zeiten zwischen Minuten und Tagen liegen. Bevorzugt liegen zwischen den Workflows Zeiten zwischen wenigen Stunden und sechs Tagen, einem halben bis sechs Tage oder ein bis 4 Tagen. Die Dauer eines Workflows kann zwischen einer Minute und 60 Minuten betragen.

Ein Workflow beinhaltet ein oder mehrere Arbeitsschritte, die unmittelbar oder kurzfristig aufeinanderfolgend durchgeführt werden müssen, wobei eine Pause zwischen zwei aufeinanderfolgenden Arbeitsschritten vorzugsweise Null ist oder oftmals einige Minuten lang sein kann, wobei kein anderer Workflow in der Zwischenzeit liegt. Ein Arbeitsschritt kann beispielsweise ein Konfluenz-Scan 11 sein, bei dem eine gemessene Konfluenz 12 ermittelt wird, um daraus eine bevorzugte Startzeit $t_p$ zu ermitteln. Weitere Arbeitsschritte können das Hinzufügen eines Abtrennmittels 14, das Suspendieren von Zellen 15, das Zentrifugieren, das Zählen lebender oder toter Zellen 17, die Zellanlage, also das Aussähen neuer Zellen 18, die Nährstoffzugabe, also das Füttern von Zellen 19 sein. Ein Workflow kann zumindest einen dieser oder einen anderen, zuvor nicht aufgeführten Arbeitsschritt beinhalten.

Die ein oder mehrere Workflows W1, W2, W3, W4, W5 aufweisenden Applikationen können periodisch über eine unbestimmte Zeit durchgeführt werden, beispielsweise zur Zelllinienpflege. Eine Applikation kann sich über mehrere Tage erstrecken.

Das System kann mehrere Instanzen derselben Applikation haben, zum Beispiel kann die Zelllinienpflege-Applikation eine erste Instanz "CHO-Zellen pflegen" oder eine zweite Instanz "Vero-Zellen pflegen" haben.

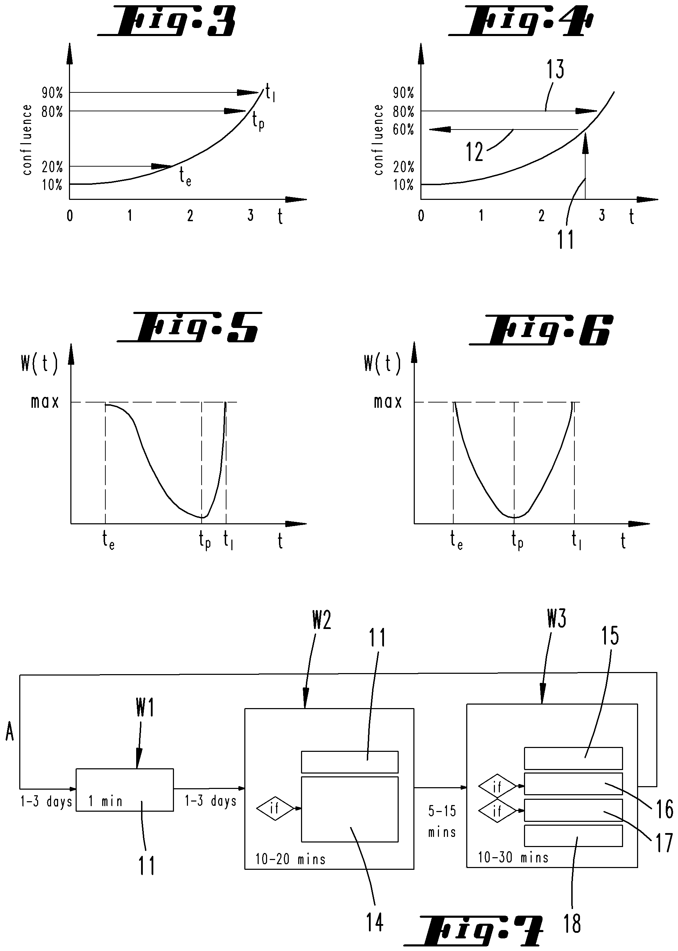

Die Figur 7 zeigt eine aus drei Workflows W1, W2, W3 bestehende Applikation A, bei der nach dem Start der Applikation nach ein bis drei Tagen ein erster Workflow W1 durchgeführt wird, bei dem ein Konfluenz-Scan 11 durchgeführt wird. Nach weiteren ein bis drei Tagen wird ein zweiter Workflow W2 durchgeführt, bei dem zunächst ebenfalls ein Konfluenz-Scan 11 durchgeführt wird und je nach Konfluenzgrad die Zellen gewaschen und ein Ablösemittel 14 zugegeben wird, um die Zellen vom Boden einer Vertiefung des Trägers abzulösen. Während der Konfluenzscan 11 etwa 1 Minute dauert, kann der Folgeschritt, zum Beispiel das Waschen oder die Zugabe von Ablösemittel 14 10 bis 20 Minuten in Anspruch nehmen. Im ersten Workflow W1 kann eine Ausgangs-Konfluenz gemessen werden. Mit der im zweiten Workflow W2 erfolgten weiteren Messung der Konfluenz kann die Teilungsrate der Zelllinie bestimmt werden. Daraus kann die bevorzugte Startzeit $t_p$ eines weiteren Workflows berechnet werden, beispielsweise wenn die voraussichtliche Konfluenz 80% beträgt.

Ein dritter Workflow W3 kann auch nach fünf bis zehn Minuten durchgeführt werden. Dieser Workflow kann die Schritte Entnahme der Zellen 15, Zentrifugieren 16, Zählen der lebenden und toten Zellen 17 und/oder das Aussähen Zellen 18 umfassen oder das Aussähen von Zellen 18 in neuen Mikroplatten 18 umfassen.

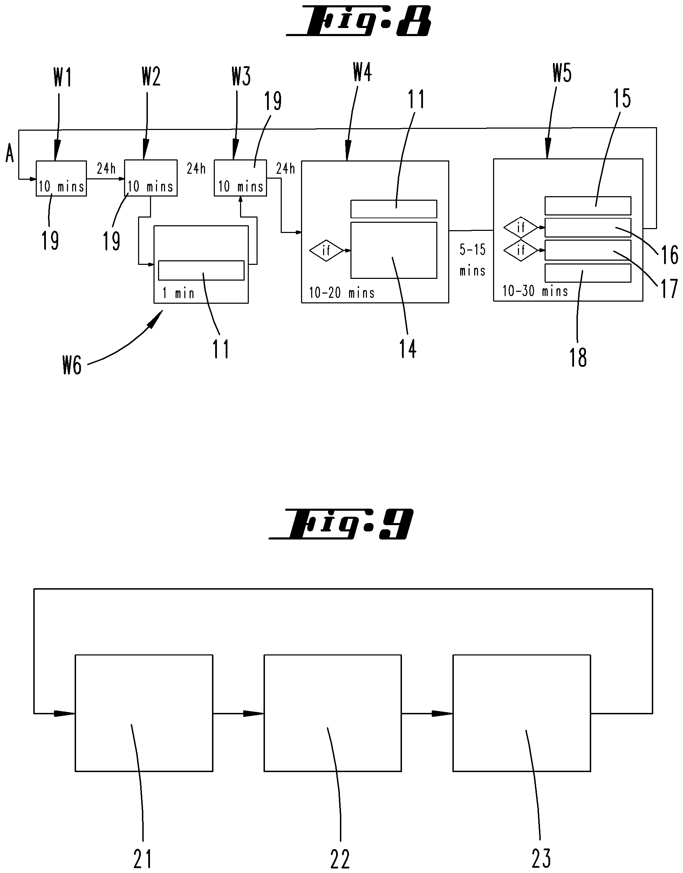

Die Figur 8 zeigt ein weiteres Ausführungsbeispiel einer Applikation, bei der fünf Workflows W1, W2, W3, W4, W5 hintereinander durchgeführt werden und die Sequenz von fünf Workflows für unbestimmte Zeit repetiert wird.

Der Workflow W1, der Workflow W2 und der Workflow W3 können jeweils eine Nährstoffzugabe 19 beinhalten. Der Workflow W6 umfasst den Schritt des Konfluenz-Scans 11. Das Intervall zwischen den Nährstoffzugaben kann zum Beispiel auf 24 Stunden festgelegt werden, und die Verdoppelungszeit der Zellen kann zwischen den Zelllinien variieren, sodass die Anzahl der Nährstoffzugaben von der Wachstumsrate der Zellen abhängt, d.h. die Anzahl der Workflows in der Applikation kann variieren. Abhängig vom Startzeitpunkt des Konfluenz-Scans 11 und von der Wachstumsrate der Zellen kann auch die Reihenfolge der Workflows W1, W2, W3 und W6 variieren (d.h. W6 kann außerhalb der in Abbildung 8 dargestellten Reihenfolge stattfinden). Der Workflow W4 umfasst den Konfluenzscan 11 und der Schritt 14 die Entfernung des Mediums und die Zugabe des Ablösemittels. Der Arbeitsablauf 5 kann die Suspendierung der Zellen im Schritt 15, das Zentrifugieren im Schritt 16, die Zählung der toten und lebenden Zellen in Schritt 17 und die Aussaat der Zellen im Schritt 18 umfassen.

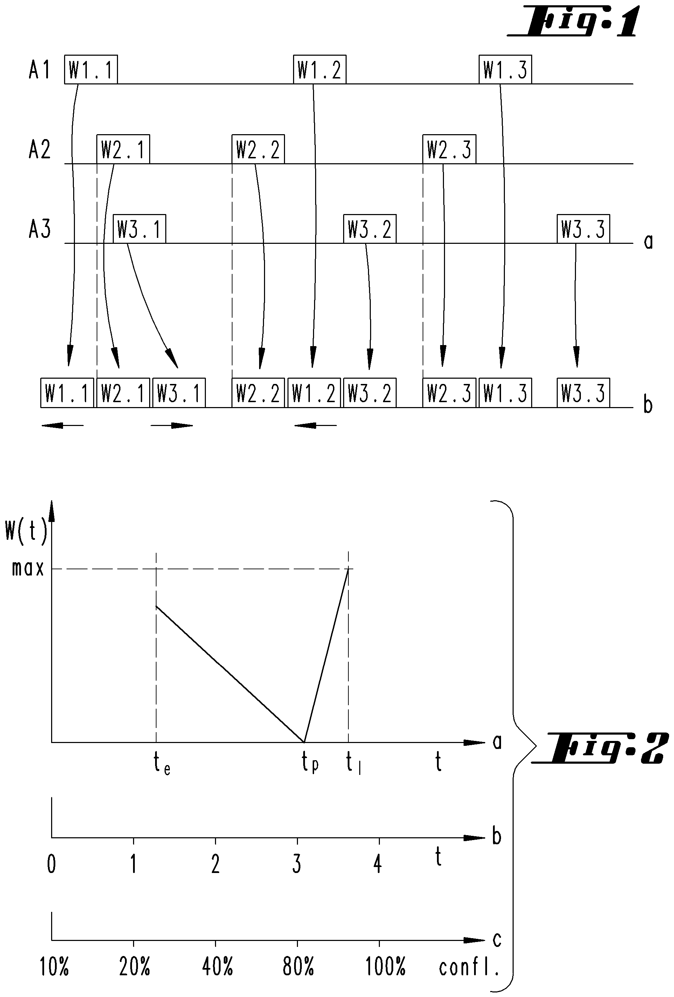

Die Figur 2 und die Figuren 5 und 6 zeigen beispielhafte Verläufe einer Wirkfunktion W(t). Die Wirkfunktion gibt einen Wert wider, der von der Startzeit t eines Workflows W1, W2, W3, W4, W5 abhängt. Die Wirkfunktion W(t) hat ein Minimum zu einer bevorzugten Startzeit $t_p$ des Workflows, dem die Wirkfunktion zugeordnet ist. Die Wirkfunktion hat ein Maximum zu einer Zeit, die der spätesten Startzeit $t_l$ beziehungsweise der frühesten Startzeit $t_e$ entspricht.

Der Abschnitt b der Figur 2 gibt beispielhaft die Anzahl von Tagen wieder, auf die sich die Startzeiten, die im Abschnitt a angegeben sind, beziehen. Der Abschnitt b der Figur 2 gibt die zugehörigen Konfluenzen, also den Bedeckungsgrad des Bodens einer Mulde, in der die Zellkultur bevorratet wird in Prozentwerten wieder. Die optimale Startzeit ist die Zeit, in der die Konfluenz voraussichtlich 80% beträgt. Die letzte Startzeit ist die Zeit, in der die Konfluenz gerade noch unter 100% liegt. Die früheste Startzeit TE ist die Zeit, in der die Konfluenz voraussichtlich etwa 20 bis 30% beträgt.

Die Steuereinrichtung 6 ist so programmiert, dass im konfliktfreien Fall jeder Workflow W1, W2, W3, W4, W5 zur bevorzugten Startzeit $t_p$ beginnt. Im Konfliktfall, in dem gemäß der durch die Applikationen A1, A2, A3 vorgegebenen Zeitpläne zwei oder mehr Workflows gleichzeitig stattfinden müssten, werden die Workflows derart verschoben, dass sie in einer Sequenz nacheinander im selben Prozessmodul 3 durchgeführt werden können. Hierzu werden die Workflows derart verschoben, dass die Summe der Wirkfunktionen ein Minimum besitzt beziehungsweise minimal ist. Eine derartige Verschiebung kann eine Änderung der Reihenfolge, d.h. eine Neuanordnung der Workflows (Arbeitsabläufe) beinhalten.

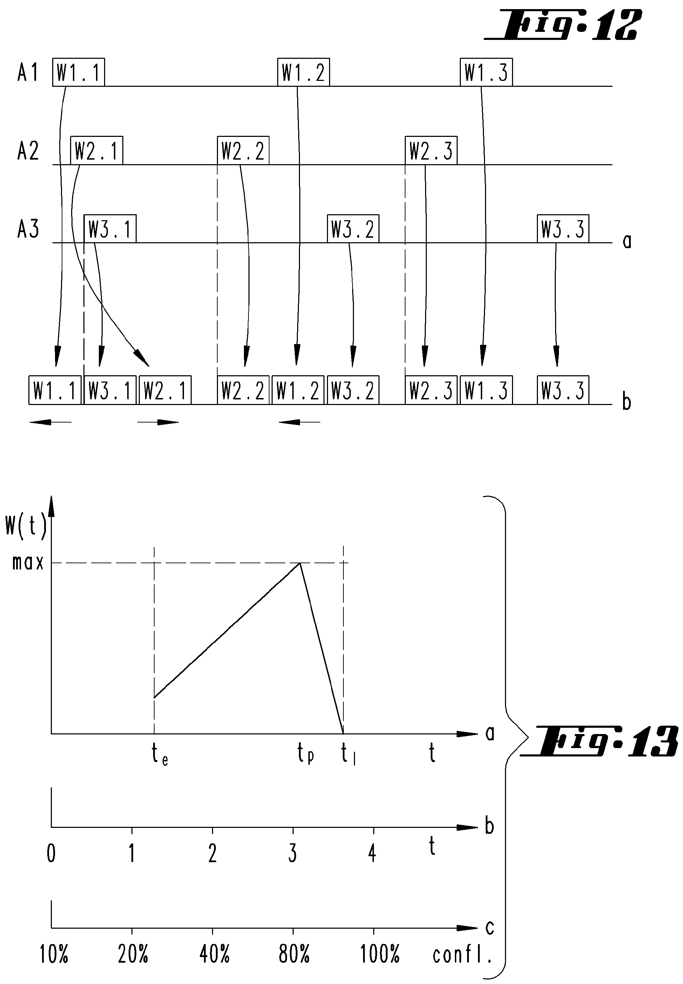

Die Figur 12 zeigt beispielhaft eine zu maximierende Wirkfunktion in einer Darstellung gemäß Figur 2.

Die Figur 1 zeigt ein Beispiel, bei dem drei Applikationen A1, A2, A3 jeweils drei Workflows W1.1, W1.2, W1.3; W2.1, W2. 2, W2.3 und W3.1, W3.2 und W3.3 aufweisen. Die Workflows W1.1, W2.1 und W3.1 der Applikationen A1, A2, A3 liegen zeitlich derart, dass sie bei einer planmäßigen Durchführung sich überlappen würden. Die Workflows W2.1, W2.2, W2.3 der Applikation A2 besitzen zudem die Besonderheit, dass sie nur zur bevorzugten Startzeit durchgeführt werden dürfen, die letzte Startzeit und die früheste Startzeit somit gewissermaßen identisch sind. Diese Workflows W2.1, W2.2 und W2.3 regieren gewissermaßen die zeitliche Anordnung der übrigen Workflows. Ein erfindungsgemäßes System kann somit solche Workflows W2.1, W2.2, W2.3 umfassen, die die zeitliche Anordnung der übrigen Workflows regieren.

Als Folge dessen wird zur Minimierung der Summe der Wirkfunktionen der Workflows W1.1, W2.1 und W3.1 der Workflow W1.1 zeitlich nach vorne und der Workflow W3.1 zeitlich nach hinten verlegt.

Die jeweils zweiten Workflows W2.2 und W3.2 brauchen beim Ausführungsbeispiel nicht verschoben zu werden, da sie planmäßig nacheinander durchgeführt werden können. Nur der Workflow W1.2 muss geringfügig nach vorne verschoben werden. Von den Workflows W1.3, W2.3 und W3.3 braucht keiner zeitlich verschoben werden. Während sich die Reihenfolge der Workflows im gezeigten Beispiel ändert, ist zu erwarten, dass eine Minimierung oder Maximierung der Summe der Wirkfunktionen in einigen Fällen dazu führen kann, dass sich die Reihenfolge der Workflows ändert.

Die Figur 11 zeigt, dass auch das Vertauschen der Workflows W3.2 und W2.1 möglich sein kann.

Die Figur 3 zeigt eine Kurve der Reproduktionsrate einer Zellkultur in der Abhängigkeit von der Zeit. Der Zeitpunkt des bevorzugten Starts eines Workflows $t_p$ ist so gewählt, dass dann die Konfluenz voraussichtlich 80% beträgt. Der Zeitpunkt des letztmöglichen Starts $t_l$ des Workflows ist so gewählt, dass dann die Konfluenz voraussichtlich 90% beträgt. Der Zeitpunkt des frühesten Starts $t_e$ des Workflows ist so gewählt, dass dann die Konfluenz voraussichtlich 20% beträgt.

Die Figur 4 zeigt den Weg einer Berechnung einer berechneten bevorzugten Startzeit 13 mithilfe einer zu einem Zeitpunkt eines Konfluenz-Scans 11 gemessenen Konfluenz 12. Zum Zeitpunkt des Konfluenz-Scans 11 wurde eine Konfluenz von 60% gemessen. Es wird der Punkt auf der Wachstumskurve ermittelt, bei dem die Konfluenz 80% beträgt.

In diesem Beispiel zählt das System beispielsweise bei einer vorherigen Durchführung eines Workflows die resuspendierten Zellen und berechnet eine Verdünnung, um eine Konfluenz von 10% der Zellen einzustellen. Ausgehend von diesen eingestellten 10% berechnet das System dann den Zeitpunkt, an dem sich die Zellen voraussichtlich in der Mitte oder am Ende eines Wachstumszyklus befinden würden (50 bis 80% Konfluenz). Aus der Anfangs-Konfluenz von beispielsweise 10% wird unter Annahme beispielsweise einer exponentiellen Wachstumskurve die Zeit berechnet, in der die Konfluenz beispielsweise 80% beträgt.

Für den Fall, dass das System die Verdopplungszeit der Zellen nicht kennt, kann es konservativ die kürzeste bekannte Verdopplungszeit für Säugetierzellen in einer Kultur oder eine Verdopplungszeit, die kleiner als die kürzeste bekannte Verdopplungszeit ist, verwenden. Zum Beispiel kann das System eine vorläufige Verdopplungszeit von 12 Stunden verwenden, um zu berechnen, wann der Konfluenz-Scan durchgeführt werden soll. Alternativ kann das System aber auch nur alle 24 Stunden einen Konfluenz-Scan durchführen, bis die Zellen eine stabile Verdopplungszeit erreicht haben. Sobald das System Daten über die Verdopplungszeit hat, kann es die Verdopplungszeit der Zellen verwenden, um zu bestimmen, wann eine 80%-ige Konfluenz erreicht ist.

Die Figuren 5 und 6 zeigen Varianten der Wirkfunktion, bei der die Wirkfunktion $W(t)$ einen nichtlinearen Verlauf besitzt. Bei der in der Figur 5 dargestellten Wirkfunktion ist das Zeitintervall zwischen der spätesten Startzeit $t_l$ und der bevorzugten Startzeit $t_p$ wesentlich kleiner, als das Zeitintervall zwischen der frühesten Startzeit $t_e$ und der bevorzugten Startzeit $t_p$.

Die Figur 6 zeigt hingegen einen bezogen auf $t_p$ symmetrischen Verlauf der Wirkfunktion $W(t)$.

Die Figur 11 zeigt als Beispiel eine grafische Oberfläche, mit der manuell der Verlauf der Wirkfunktion $W(t)$ durch "Ziehen" von Ankerpunkten auf einem Bildschirm voreingestellt werden kann.

Es kann erwünscht sein, dass Benutzer dem System Applikationen, Workflows oder andere Arbeitsabläufe hinzufügen können, was ein weiteres anspruchsvolles, vom System zu bewältigendes Planungsproblem darstellen kann. Es können Zeiten eines Zeitplans geändert werden. Es kann insbesondere berücksichtigt werden, dass einige Zelllinien schneller oder langsamer wachsen als erwartet. Während eines Workflows können ein oder mehrere Trägerplatten unmittelbar hintereinander mit der Behandlungseinrichtung 4 behandelt werden. Dies erfolgt bevorzugt mit denselben Arbeitsschritten.

Erfindungsgemäß wird ein Zellkultursystem bereitgestellt. In einer Implementierung hat das Zellkultursystem einen Inkubator, einen Kühlschrank, zwei Lager für Laborgeräte bei Raumtemperatur und ein Prozessmodul 3. Das Prozessmodul 3 kann einen Flüssigkeits-Handhaber, beispielsweise eine Pipette aufweisen. Andere Mechanismen, wie beispielsweise einen Entkapper zum Entkappen von Flaschen, einen Greifer zum Handhaben von Trägern 1 können ebenso wie ein Mikroskop vorgesehen sein. Abhängig von den Applikationen und Workflows oder anderen Arbeitsabläufen kann die Kapazität des Systems entweder durch die Kapazität des Inkubators für die Träger, insbesondere Trägerplatten und insbesondere die Anzahl der Platten, die der Inkubator verarbeiten kann, oder durch die Kapazität (Geschwindigkeit) des Prozessmoduls begrenzt werden. Es ist somit nur eine begrenzte Anzahl der Arbeitsabläufe beziehungsweise Workflows in einem Prozessmodul 3 möglich.

Das System kann Applikationen ausführen, die beispielsweise die Pflege von Zelllinien oder einen zellbasierten "Assay" oder ein Experiment umfassen. Die Applikationen können für eine unbestimmte Zeit in Bezug auf die Zelllinienpflege laufen und sich insbesondere über Tage oder Wochen erstrecken.

Die Workflows umfassen insbesondere den "Konfluenz-Scan", das Hinzufügen eines "Detachmentagent" und die Zellentnahme. Der Workflow Zellentnahme kann einzelne Operationen enthalten, nämlich die Entnahme von Zellen und das Aussähen der Zellen. Ferner können Arbeitsschritte, wie das Zentrifugieren oder das Zählen von toten und lebendigen Zellen vorgesehen sein. Für die Kultivierung einer bestimmten Zelllinie, z.B. CHO-Zellen, wird eine Instanz der Applikation erstellt, die die Parameter für die Kultivierung dieser Zelllinie enthält. Parameter können das komplette Medium-Rezept sein, die Passagen-Konfluenz, das Splitverhältnis oder die Anzahl der Träger, beispielsweise Trägerplatten wie Mikroplatten.

Die Arbeitsschritte innerhalb eines Workflows sind zusammenhängend und haben keine Lücken und insbesondere keine Lücken von mehr als einer Minute zwischen zwei Arbeitsschritten. Die Zeit zwischen zwei Workflows in derselben Applikation ist größer als eine Minute und beträgt typischerweise Minuten, Stunden oder Tage. Der Transport eines Trägers aus dem Aufbewahrungsmodul 2 zum Prozessmodul 3 und eine dortige Behandlung kann verzögert werden, wenn die hierzu erforderliche Konfluenz noch nicht erreicht wird. Ein Workflow kann somit Arbeitsschritte beinhalten, die optional durchgeführt werden, beispielsweise wenn ein Konfluenz-Scan einen bestimmten Wert erreicht. Wird im Konfluenz-Scan der bestimmte Wert nicht erreicht, kann ein oder können mehrere folgende Arbeitsschritte unterlassen oder zurückgestellt werden.

Das dem erfindungsgemäßen System zugrunde liegende Verfahren ist in der Lage, einen Zeitplan zu verarbeiten, bei dem die Dauer und die Intervalle der Workflows voneinander abweichen. Das Verfahren lässt sich wie folgt unter Verweis auf die Figur 9 zusammenfassen:

Definieren eines Workflows zu einem bevorzugten Startpunkt $t_p$, eine früheste Startzeit $t_e$ und eine späteste Startzeit $t_l$ sowie die Kosten für die Durchführung des Workflows zu einer anderen Startzeit als $t_p$, wobei die Kosten in der Wirkfunktion $W(t)$ dargestellt sind (Durchführung des Workflows 21).

Wenn eine Eingabe durch einen Benutzer erfolgt ist oder wenn Daten gefunden werden, die z.B. die bevorzugte Startzeit eines nachfolgenden Workflows ändern würde, dann werden die Daten (Startzeiten) aller nachfolgenden Workflows neu berechnet (22). Die Berechnung erfolgt derart, dass eine Summe, insbesondere eine Gesamtsumme der Wirkfunktionen der Workflows ein Minimum einnehmen (23). Das Minimum muss kein globales Minimum sein, es kann ein lokales Minimum sein.

Die Summe Σ aller Wirkfunktionen W(t) aller Workflows kann als mehrdimensionale Funktion dargestellt werden.

$$\Sigma = W_1(t_1) + W_2(t_2) + \ldots + W_n(t_n)$$

Von der durch Verschieben der jeweiligen Startzeiten und unter Beachtung der zeitlichen Dauer eines jeden Workflows derart ein globales oder lokales Minimum berechnet werden kann

$$\frac{d}{dt}\,\Sigma = 0$$

das der Randbedingung unterliegt, dass alle Workflows zeitlich aufeinander folgen und der Startzeitpunkt jedes der Workflows in keiner Zeitdauer eines anderen Workflows liegt.

Die Erfindung liefert ein Verfahren zum Mischen der Workflows, bei dem eine Summe von Wirkfunktionen W(t) minimiert wird, die beispielsweise die für die Durchführung der Workflows aufzuwendenden Kosten darstellt, sodass das erfindungsgemäße Verfahren ein kostenoptimiertes Verfahren ist.

Die Erfindung betrifft somit einen Planer oder eine Plansoftware, mit der eine Vielzahl von Applikationen (Anwendungen) verwaltet werden können, wobei jede Anwendung ein oder mehrere Workflows (Arbeitsabläufe) beinhaltet. Die Arbeitsabläufe der verschiedenen Anwendungen werden optimiert durchgeführt, wobei die zuvor beschriebenen Optimierungscharaktere verwendet werden.

Die vorstehenden Ausführungen dienen der Erläuterung der von der Anmeldung insgesamt erfassten Erfindungen, die den Stand der Technik zumindest durch die folgenden Merkmalskombinationen jeweils auch eigenständig weiterbilden, wobei zwei, mehrere oder alle dieser Merkmalskombinationen auch kombiniert sein können, nämlich:

Ein System, das dadurch gekennzeichnet ist, dass jedem Workflow W1.1, W1.2, W1.3; W2.1, W2.2, W2.3; W3.1, W3.2, W3.3 eine von der Startzeit t abhängige Wirkfunktion W(t) zugeordnet ist und die Reihenfolge und die Startzeiten (t) der Workflows W1.1, W1.2, W1.3; W2.1, W2.2, W2.3; W3.1, W3.2, W3.3 verschiedener Applikationen A1, A2, A3 durch eine Minimierung der Summe Σ der Wirkfunktionen W(t) berechnet wird.

Ein Verfahren, das dadurch gekennzeichnet ist, dass jedem Workflow W1.1, W1.2, W1.3; W2.1, W2.2, W2.3; W3.1, W3.2, W3.3 eine von der Startzeit t abhängige Wirkfunktion W(t) zugeordnet ist und die Reihenfolge und die Startzeiten (t) der Workflows W1.1, W1.2, W1.3; W2.1, W2.2, W2.3; W3.1, W3.2, W3.3 verschiedener Applikationen A1, A2, A3 oder Instanzen derselben Applikation durch eine Minimierung der Summe Σ der Wirkfunktionen W(t) berechnet wird.

Ein System oder ein Verfahren, das dadurch gekennzeichnet ist, dass jedem Workflow W1.1, W1.2, W1.3; W2.1, W2.2, W2.3; W3.1, W3.2, W3.3 eine bevorzugte Startzeit $t_p$ zugeordnet ist, bei der die dem Workflow W1.1, W1.2, W1.3; W2.1, W2.2, W2.3; W3.1, W3.2, W3.3 zugeordnete Wirkfunktion W(t) ein Minimum aufweist.

Ein System oder ein Verfahren, das dadurch gekennzeichnet ist, dass zumindest einige erste Workflows W1.1, W1.2, W1.3 der verschiedenen Applikationen A1, A2, A3 eine erste bevorzugte Startzeit $t_p$ aufweisen, die in eine Behandlungsdauer eines zweiten Workflows fallen, dessen zweite bevorzugte Startzeit $t_p$ vor der ersten bevorzugten Startzeit $t_p$ liegt, wobei die Steuereinrichtung 6 die erste und/oder die zweite Startzeit derart zeitlich verschiebt, dass die beiden ersten und zweiten Workflow W1.1, W1.2, W1.3; W2.1, W2.2, W2.3; W3.1, W3.2, W3.3 nacheinander durchgeführt werden, wobei die ersten und zweiten Startzeiten so gewählt werden, dass die Summe Σ der Wirkfunktionen W(t) minimimiert oder maximiert ist.

Ein System oder ein Verfahren, das dadurch gekennzeichnet ist, dass zumindest einigen der Workflows W2.1, W2.2, W2.3 eine früheste Startzeit $t_e$ zugeordnet ist, vor der der Workflow W.1, W2.2, W2.3 nicht gestattet werden darf und/oder eine späteste Startzeit $t_l$ zugeordnet ist, nach der der Workflow W2.1, W2.2, W2.3 nicht gestattet werden darf, wobei insbesondere vorgesehen ist, dass die Wirkfunktion W(t) bei der frühesten Startzeit $t_e$ und bei der spätesten Startzeit $t_l$ ein Maximum aufweist.

Ein System oder ein Verfahren, das dadurch gekennzeichnet ist, dass die Wirkfunktion W(t) einen von einer frühesten Startzeit $t_e$ linearer oder nicht linearer zur bevorzugten Startzeit $t_p$ abfallenden oder ansteigenden und einen von der bevorzugten Startzeit $t_p$ zur spätesten Startzeit $t_l$ linearer oder nicht linearer ansteigenden oder absteigenden Ast aufweist.

Ein System oder ein Verfahren, das dadurch gekennzeichnet ist, dass der Verlauf der Wirkfunktion W(t) derart gewählt ist, dass die für die Durchführung der Applikation A1, A2, A3 aufzuwendenden Kosten beim Start der Workflows W1.1, W1.2, W1.3; W2.1, W2.2, W2.3; W3.1, W3.2, W3.3 zu den bevorzugten Startzeiten $t_p$ ein Minimum und zur frühesten und spätesten Startzeit jeweils ein Maximum besitzen.

Ein System oder ein Verfahren, das dadurch gekennzeichnet ist, dass zumindest einige der Workflows W1.1, W2.1, W3.1 einen das Zellwachstum überwachenden Überwachungsschritt aufweisen, in dem die Konfluenz einer Zellkultur ermittelt wird, und abhängig davon die bevorzugte Startzeit $t_p$ zumindest eines darauffolgenden Workflows W1.1, W2.2, W3.3 festgelegt wird und/oder dass im Überwachungsschritt die früheste Startzeit $t_l$ und/oder die späteste Startzeit $t_l$ eines folgenden Workflows W1.2, W2.2, W3.2 festgelegt wird.

Ein System oder ein Verfahren, das dadurch gekennzeichnet ist, dass der Workflow W1.1, W1.2, W1.3; W2.1, W2.2, W2.3; W3.1, W3.2, W3.3 zumindest ein oder mehrere der folgenden Arbeitsschritte umfasst: Anlegen einer Zellkultur 18, Zählen der lebenden oder toten Zellen 17, Entnahme von Zellen der Zellkultur, Lösen der Zellen vom Boden einer Mulde des Trägers 1, Hinzufügen eines Abtrennmittels 14, Konfluenz-Scan 11, Nährstoffzugabe 19 oder Aufgabe der Zellkultur 15 oder Sähen von Zellen.

Ein System oder ein Verfahren, das dadurch gekennzeichnet ist, dass die Transporteinrichtung 5 einen ersten Träger 1 zur Durchführung eines ersten Workflows W1.1 derart zu einer ersten Zeit einem Lagerplatz im Aufbewahrungsmodul 2 entnimmt und zum Prozessmodul 3 transportiert, dass die Behandlungseinrichtung 4 die vom ersten Träger getragene Zellkultur zu einer ersten Startzeit t zu behandeln beginnt und die Transporteinrichtung 5 nachfolgend einen zweiten Träger 1 zur Durchführung eines zweiten Workflows 2.1 derart zu einer zweiten Zeit einem Lagerplatz im Aufbewahrungsmodul 2 entnimmt und zum Prozessmodul 3 transportiert, dass die Behandlungseinrichtung 4 die vom zweiten Träger 1 getragene Zellkultur nach Beendigung der Durchführung des ersten Workflows W1.1 zu einer zweiten Startzeit t zu behandeln beginnt, wobei die beiden Startzeiten t derart berechnet sind, dass die Summe $\Sigma$ der Wirkfunktionen $W(t)$ minimiert oder maximiert ist.

Ein System oder ein Verfahren, das gekennzeichnet ist durch eine grafische Dateneingabeeinrichtung, mit der ein Benutzer den Verlauf einer Wirkfunktion vorgeben kann.

Ein System oder ein Verfahren, das dadurch gekennzeichnet ist, dass die Behandlungseinrichtung 4 eine Pipettiereinrichtung, ein Mikroskop und/oder eine Öffnungseinrichtung zum Öffnen von Nährmittel enthaltenden Behältern aufweist und/oder dass das Aufbewahrungsmodul 2 ein Inkubator ist und/oder dass die Transporteinrichtung 5 einen Greifarm oder Endeffektor aufweist und/oder automatisiert ist.

Ein System oder ein Verfahren, das dadurch gekennzeichnet ist, dass das Minimum oder Maximum ein lokales Minimum oder Maximum ist und/ oder dass das Minimum oder Maximum ein über die Dauer einer beschränkten Anzahl von Workflows W1.1, W1.2, W1.3; W2.1, W2.2, W2.3; W3.1, W3.2, W3.3 berechneter Wert ist.

Alle offenbarten Merkmale sind (für sich, aber auch in Kombination untereinander) erfindungswesentlich. In die Offenbarung der Anmeldung wird hiermit auch der Offenbarungsinhalt der zugehörigen/beigefügten Prioritätsunterlagen (Abschrift der Voranmeldung) vollinhaltlich mit einbezogen, auch zu dem Zweck, Merkmale dieser Unterlagen in Ansprüche vorliegender Anmeldung mit aufzunehmen. Die Unteransprüche charakterisieren, auch ohne die Merkmale eines in Bezug genommenen Anspruchs, mit ihren Merkmalen eigenständige erfinderische Weiterbildungen des Standes der Technik, insbesondere um auf Basis dieser Ansprüche Teilanmeldungen vorzunehmen. Die in jedem Anspruch angegebene Erfindung kann zusätzlich ein oder mehrere der in der vorstehenden Beschreibung, insbesondere mit Bezugsziffern versehene und/oder in der Bezugsziffernliste angegebene Merkmale aufweisen. Die Erfindung betrifft auch Gestaltungsformen, bei denen einzelne der in der vorstehenden Beschreibung genannten Merkmale nicht verwirklicht sind, insbesondere soweit sie erkennbar für den jeweiligen Verwendungszweck entbehrlich sind oder durch andere technisch gleichwirkende Mittel ersetzt werden können.

### Liste der Bezugszeichen

| | | | | |
|---|---|---|---|---|
| 1 | Träger | | W1.1 | Workflow |
| 2 | Aufbewahrungsmodul | | W1.2 | Workflow |
| 3 | Prozessmodul | | W1.3 | Workflow |
| 4 | Behandlungseinrichtung | | W2.1 | Workflow |
| 5 | Transporteinrichtung | | W2.2 | Workflow |
| 6 | Steuereinrichtung | | W2.3 | Workflow |
| 7 | Speichereinrichtung | | W3.1 | Workflow |
| 11 | Konfluenz-Scan | | W3.2 | Workflow |
| 12 | gemessene Konfluenz | | W3.3 | Workflow |
| 13 | berechnete bevorzugte Startzeit | | W1 | Workflow |
| | | | W2 | Workflow |
| 14 | Hinzufügen eines Ablösemittels | | W3 | Workflow |
| | | | W4 | Workflow |
| 15 | Suspendieren der Zellen | | W5 | Workflow |
| 16 | Zentrifugieren | | W(t) | Wirkfunktion |
| 17 | Zählen | | | |
| 18 | Zellaussähen | | t | Startzeit |

(fortgesetzt)

| 19 | Nährstoffzugabe | $t_e$ | früheste Startzeit |
|---|---|---|---|
| 21 | Durchführung eines Workflows | $t_p$ | bevorzugte Startzeit |
| | | $t_l$ | späteste Startzeit |
| 22 | Neuberechnen von $t_e$, $t_p$, $t_l$ | | |
| 23 | zeitliches Verschieben | $\Sigma$ | Summe |
| | | | |
| A | Applikation | | |
| A1 | Applikation | | |
| A2 | Applikation | | |
| A3 | Applikation | | |

**Patentansprüche**

1. Verfahren zum Kultivieren biologischer Zellkulturen, bei dem eine Vielzahl von Trägern (1) jeweils ein oder mehrere Zellkulturen tragen, die in mindestens einem Aufbewahrungsmodul (2) aufbewahrt werden, wobei die Zellkulturen in einem Prozessmodul (3) mittels zumindest einer Behandlungseinrichtung (4) behandelt werden, wobei die Träger (1) mit einer Transporteinrichtung (5) zwischen dem Aufbewahrungsmodul (2) und dem Prozessmodul (3) transportiert werden, wobei mit einer Steuereinrichtung (6), die eine Speichereinrichtung (7) aufweist die Transporteinrichtung (5) und die Behandlungseinrichtung (4) gesteuert wird und in der Speichereinrichtung (7) mehreren voneinander verschiedenen Zellkulturen zugeordnete Applikationen (A1, A2, A3) gespeichert sind, wobei jede Applikation (A1, A2, A3) eine Behandlungsvorschrift zur Behandlung der zugeordneten Zellkultur ist, die ein oder mehrere Workflows (W1.1, W1.2, W1.3; W2.1, W2.2, W2.3; W3.1, W3.2, W3.3) beinhaltet, die in zeitlichen Abständen nacheinander jeweils zu einer Startzeit (t) in dem Prozessmodul (3) durchgeführt werden, wobei ein Workflow (W1.1, W1.2, W1.3; W2.1, W2.2, W2.3; W3.1, W3.2, W3.3) einen oder mehrere kurzfristig aufeinanderfolgend durchzuführende Arbeitsschritte beinhaltet, wobei die Steuereinrichtung (6) die gleichzeitige Durchführung einer Mehrzahl von Applikationen (A1, A2, A3) steuert und dabei die ein oder mehreren Workflows (W1.1, W1.2, W1.3; W2.1, W2.2, W2.3; W3.1, W3.2, W3.3) der voneinander verschiedenen Applikationen (A1, A2, A3) in einer zeitlichen Reihenfolge hintereinander im selben Prozessmodul (3) startet, **dadurch gekennzeichnet, dass** jedem Workflow (W1.1, W1.2, W1.3; W2.1, W2.2, W2.3; W3.1, W3.2, W3.3) eine von der Startzeit (t) abhängige Wirkfunktion (W(t)) zugeordnet ist und die Reihenfolge und die Startzeiten (t) der Workflows (W1.1, W1.2, W1.3; W2.1, W2.2, W2.3; W3.1, W3.2, W3.3) verschiedener Applikationen (A1, A2, A3) oder Instanzen derselben Applikation durch eine Minimierung oder Maximierung der Summe ($\Sigma$) der Wirkfunktionen (W(t)) berechnet wird.

2. Verfahren nach Anspruch 1, **dadurch gekennzeichnet, dass** jedem Workflow (W1.1, W1.2, W1.3; W2.1, W2.2, W2.3; W3.1, W3.2, W3.3) eine bevorzugte Startzeit ($t_p$) zugeordnet ist, bei der die dem Workflow (W1.1, W1.2, W1.3; W2.1, W2.2, W2.3; W3.1, W3.2, W3.3) zugeordnete Wirkfunktion (W(t)) ein Minimum oder Maximum aufweist.

3. Verfahren nach Anspruch 2, **dadurch gekennzeichnet, dass** zumindest einige erste Workflows (W1.1, W1.2, W1.3) der verschiedenen Applikationen (A1, A2, A3) eine erste bevorzugte Startzeit ($t_p$) aufweisen, die in eine Behandlungsdauer eines zweiten Workflows fallen, dessen zweite bevorzugte Startzeit ($t_p$) vor der ersten bevorzugten Startzeit ($t_p$) liegt, wobei die Steuereinrichtung (6) die erste und/oder die zweite Startzeit derart zeitlich verschiebt, dass die beiden ersten und zweiten Workflow (W1.1, W1.2, W1.3; W2.1, W2.2, W2.3; W3.1, W3.2, W3.3) nacheinander durchgeführt werden, wobei die ersten und zweiten Startzeiten so gewählt werden, dass die Summe ($\Sigma$) der Wirkfunktionen (W(t)) minimiert oder maximiert ist.

4. Verfahren nach einem der Ansprüche 2 oder 3, **dadurch gekennzeichnet, dass** zumindest einigen der Workflows (W2.1, W2.2, W2.3) eine früheste Startzeit ($t_e$) zugeordnet ist, vor der der Workflow (W2.1, W2.2, W2.3) nicht gestattet werden darf und/oder eine späteste Startzeit ($t_l$) zugeordnet ist, nach der der Workflow (W2.1, W2.2, W2.3) nicht gestattet werden darf, wobei insbesondere vorgesehen ist, dass die Wirkfunktion (W(t)) bei der frühesten Startzeit ($t_e$) und bei der spätesten Startzeit ($t_l$) ein Maximum aufweist.

5. Verfahren nach einem der Ansprüche 2 bis 4, **dadurch gekennzeichnet, dass** die Wirkfunktion (W(t)) einen von einer frühesten Startzeit ($t_e$) linearer oder nicht linearer zur bevorzugten Startzeit ($t_p$) abfallenden oder ansteigenden und einen von der bevorzugten Startzeit ($t_p$) zur spätesten Startzeit ($t_l$) linearer oder nicht linearer ansteigenden oder ansteigenden Ast aufweist.

6. Verfahren nach einem der vorhergehenden Ansprüche, **dadurch gekennzeichnet, dass** der Verlauf der Wirkfunktion (W(t)) derart gewählt ist, dass die für die Durchführung der Applikation (A1, A2, A3) aufzuwendenden Kosten beim Start der Workflows (W1.1, W1.2, W1.3; W2.1, W2.2, W2.3; W3.1, W3.2, W3.3) zu den bevorzugten Startzeiten ($t_p$) ein Minimum und zur frühesten und spätesten Startzeit jeweils ein Maximum besitzen.

7. Verfahren nach einem der vorhergehenden Ansprüche, **dadurch gekennzeichnet, dass** zumindest einige der Workflows (W1.1, W2.1, W3.1) einen das Zellwachstum überwachenden Überwachungsschritt aufweisen, in dem die Konfluenz einer Zellkultur ermittelt wird, und abhängig davon die bevorzugte Startzeit ($t_p$) zumindest eines darauffolgenden Workflows (W1.1, W2.2, W3.3) festgelegt wird und/oder dass im Überwachungsschritt die früheste Startzeit ($t_l$) und/oder die späteste Startzeit ($t_l$) eines folgenden Workflows (W1.2, W2.2, W3.2) festgelegt wird.

8. Verfahren nach einem der vorhergehenden Ansprüche, **dadurch gekennzeichnet, dass** der Workflow (W1.1, W1.2, W1.3; W2.1, W2.2, W2.3; W3.1, W3.2, W3.3) zumindest ein oder mehrere der folgenden Arbeitsschritte umfasst: Anlegen einer Zellkultur (18), Zählen der lebenden oder toten Zellen (17), Entnahme von Zellen der Zellkultur, Lösen der Zellen vom Boden einer Mulde des Trägers (1), Hinzufügen eines Abtrennmittels 14, Konfluenz-Scan (11), Nährstoffzugabe (19), Aufgabe der Zellkultur (15) oder Sähen von Zellen.

9. System zum Kultivieren biologischer Zellkulturen, mit einer Vielzahl von Trägern (1), die jeweils ein oder mehrere Zellkulturen tragen, mit mindestens einem Aufbewahrungsmodul (2), in dem die Träger (1) aufbewahrt werden, mit einem Prozessmodul (3), in dem die von den Trägern (1) getragenen Zellkulturen mittels zumindest einer Behandlungseinrichtung (4) behandelt werden, mit einer Transporteinrichtung (5), mit der die Träger (1) zwischen dem Aufbewahrungsmodul (2) und dem Prozessmodul (3) transportiert werden und mit einer Steuereinrichtung (6), die eine Speichereinrichtung (7) aufweist und die die Transporteinrichtung (5) und die Behandlungseinrichtung (4) steuert, wobei in der Speichereinrichtung (7) mehreren voneinander verschiedenen Zellkulturen zugeordnete Applikationen (A1, A2, A3) gespeichert sind, wobei jede Applikation (A1, A2, A3) eine Behandlungsvorschrift zur Behandlung der zugeordneten Zellkultur ist, die ein oder mehrere Workflows (W1.1, W1.2, W1.3; W2.1, W2.2, W2.3; W3.1, W3.2, W3.3) beinhaltet, die in zeitlichen Abständen nacheinander jeweils zu einer Startzeit (t) in dem Prozessmodul (3) durchgeführt werden, wobei ein Workflow (W1.1, W1.2, W1.3; W2.1, W2.2, W2.3; W3.1, W3.2, W3.3) einen Arbeitsschritt beinhaltet oder mehrere kurzfristig aufeinanderfolgend durchzuführende Arbeitsschritte, wobei die Steuereinrichtung (6) die gleichzeitige Durchführung einer Mehrzahl von Applikationen (A1, A2, A3) steuert, dabei die ein oder mehreren Workflows (W1.1, W1.2, W1.3; W2.1, W2.2, W2.3; W3.1, W3.2, W3.3) der voneinander verschiedenen Applikationen (A1, A2, A3) in einer zeitlichen Reihenfolge hintereinander im selben Prozessmodul (3) startet, **dadurch gekennzeichnet, dass** jedem Workflow (W1.1, W1.2, W1.3; W2.1, W2.2, W2.3; W3.1, W3.2, W3.3) eine von der Startzeit (t) abhängige Wirkfunktion (W(t)) zugeordnet ist und die Reihenfolge und die Startzeiten (t) der Workflows (W1.1, W1.2, W1.3; W2.1, W2.2, W2.3; W3.1, W3.2, W3.3) verschiedener Applikationen (A1, A2, A3) oder Instanzen derselben Applikation durch eine Minimierung oder Maximierung der Summe ($\Sigma$) der Wirkfunktionen (W(t)) berechnet wird.

10. System nach Anspruch 9, **dadurch gekennzeichnet, dass** die Steuereinrichtung 6 zur Durchführung eines Verfahrens gemäß einem der Ansprüche 1 bis 8 programmiert ist.

11. System nach Anspruch 9 oder 10, **dadurch gekennzeichnet, dass** die Transporteinrichtung (5) einen ersten Träger (1) zur Durchführung eines ersten Workflows (W1.1) derart zu einer ersten Zeit einem Lagerplatz im Aufbewahrungsmodul (2) entnimmt und zum Prozessmodul (3) transportiert, dass die Behandlungseinrichtung (4) die vom ersten Träger getragene Zellkultur zu einer ersten Startzeit (t W1.1) zu behandeln beginnt und die Transporteinrichtung (5) nachfolgend einen zweiten Träger (1) zur Durchführung eines zweiten Workflows (W2.1) derart zu einer zweiten Zeit einem Lagerplatz im Aufbewahrungsmodul (2) entnimmt und zum Prozessmodul (3) transportiert, dass die Behandlungseinrichtung (4) die vom zweiten Träger (1) getragene Zellkultur nach Beendigung der Durchführung des ersten Workflows (W1.1) zu einer zweiten Startzeit (t) zu behandeln beginnt, wobei die beiden Startzeiten (t) derart berechnet sind, dass die Summe ($\Sigma$) der Wirkfunktionen (W(t)) minimiert oder maximiert ist.

12. System nach einem der Ansprüche 9 bis 11, **gekennzeichnet durch** eine grafische Dateneingabeeinrichtung, mit der ein Benutzer den Verlauf einer Wirkfunktion vorgeben kann.

13. System nach einem der Ansprüche 9 bis 12, **dadurch gekennzeichnet, dass** die Behandlungseinrichtung (4) eine Pipettiereinrichtung, ein Mikroskop und/oder eine Öffnungseinrichtung zum Öffnen von Nährmittel enthaltenden Behältern aufweist und/oder dass das Aufbewahrungsmodul (2) ein Inkubator ist und/oder dass die Transporteinrichtung (5) einen Greifarm oder Endeffektor aufweist und/oder automatisiert ist.

14. System nach einem der Ansprüche 9 bis 13, **dadurch gekennzeichnet, dass** das Minimum oder Maximum ein lokales Minimum oder Maximum ist und/oder dass das Minimum oder Maximum ein über die Dauer einer beschränkten Anzahl von Workflows (W1.1, W1.2, W1.3; W2.1, W2.2, W2.3; W3.1, W3.2, W3.3) berechneter Wert ist.

15. Software für ein System gemäß einem der Ansprüche 9 bis 14, die programmiert ist zur Durchführung eines Verfahrens gemäß einem der Ansprüche 1 bis 8.

**Claims**

1. A method for cultivating biological cell cultures, in which a plurality of carriers (1) each carry one or several cell cultures, which are stored in at least one storage module (2), wherein the cell cultures are treated in a process module (3) by means of at least one treatment device (4), wherein the carriers (1) are transported with a transport device (5) between the storage module (2) and the process module (3), wherein a control device (6) having a memory device (7) is used to control the transport device (5) and the treatment device (4), and applications (A1, A2, A3) assigned to different cell cultures are stored in the memory device (7), wherein each application (A1, A2, A3) is a treatment specification for treating the assigned cell culture, which contains one or several workflows (W1.1, W1.2, W1.3; W2.1, W2.2, W2.3; W3.1, W3.2, W3.3), which are performed at time intervals successively at a respective start time (t) in the process module (3), wherein a workflow (W1.1, W1.2, W1.3; W2.1, W2.2, W2.3; W3.1, W3.2, W3.3) contains one work step or several work steps to be performed one after the other at short notice, wherein the control device (6) controls the simultaneous performance of a plurality of applications (A1, A2, A3), and thereby starts one or several workflows (W1.1, W1.2, W1.3; W2.1, W2.2, W2.3; W3.1, W3.2, W3.3) of the different applications (A1, A2, A3) in chronological sequence one after the other in the same process module (3), **characterized in that** each workflow (W1.1, W1.2, W1.3; W2.1, W2.2, W2.3; W3.1, W3.2, W3.3) has assigned to it an active function W(t) that depends on the start time (t), and the sequence and start times (t) of the workflows (W1.1, W1.2, W1.3; W2.1, W2.2, W2.3; W3.1, W3.2, W3.3) of different applications (A1, A2, A3) or instances of the same application are calculated by minimizing or maximizing the sum ($\Sigma$) of active functions (W(t)).

2. The method according to claim 1, **characterized in that** each workflow (W1.1, W1.2, W1.3; W2.1, W2.2, W2.3; W3.1, W3.2, W3.3) has assigned to it a preferred start time ($t_p$), at which the active function (W(t)) assigned to the workflow (W1.1, W1.2, W1.3; W2.1, W2.2, W2.3; W3.1, W3.2, W3.3) has a minimum or maximum.

3. The method according to claim 2, **characterized in that** at least some first workflows (W1.1, W1.2, W1.3) of the different applications (A1, A2, A3) have a first preferred start time ($t_p$), which fall within a treatment duration of a second workflow whose second preferred start time ($t_p$) lies before the first preferred start time ($t_p$), wherein the control device (6) shifts the first and/or second start time in such a way that the two first and second workflows (W1.1, W1.2, W1.3; W2.1, W2.2, W2.3; W3.1, W3.2, W3.3) are performed one after the other, wherein the first and second start times are selected in such a way as to minimize or maximize the sum ($\Sigma$) of active functions (W(t)).

4. The method according to one of claims 2 or 3, **characterized in that** at least some of the workflows (W2.1, W2.2, W2.3) have allocated to them an earliest start time ($t_e$) before which the workflow (W2.1, W2.2, W2.3) must not be started and/or a latest start time ($t_l$) after which the workflow (W2.1, W2.2, W2.3) must not be started, wherein it is provided in particular that the active function (W(t)) has a maximum at the earliest start time ($t_e$) and at the latest start time ($t_l$).

5. The method according to one of claims 2 to 4, **characterized in that** the active function (W(t)) has a branch that linearly or nonlinearly descends or ascends from an earliest start time ($t_e$) to the preferred start time ($t_p$) and a branch that linearly or nonlinearly ascends or descends from the preferred start time ($t_p$) to the latest start time ($t_l$).

6. The method according to one of the preceding claims, **characterized in that** the progression of the active function (W(t)) is selected in such a way that the costs to be expended for performing the application (A1, A2, A3) at the start of the workflows (W1.1, W1.2, W1.3; W2.1, W2.2, W2.3; W3.1, W3.2, W3.3) have a minimum at the preferred start times ($t_p$) and a respective maximum at the earliest and latest start times.

7. The method according to one of the preceding claims, **characterized in that** at least some of the workflows (W1.1, W2.1, W3.1) have a monitoring step that monitors cell growth, in which the confluence of a cell culture is determined, and the preferred start time ($t_p$) of at least one ensuing workflow (W1.1, W2.2, W3.3) is determined depending thereupon, and/or that the earliest start time ($t_e$) and/or the latest start time ($t_l$)of an ensuing workflow (W1.2, W2.2, W3.2) is determined in the monitoring step.

8. The method according to one of the preceding claims, **characterized in that** the workflow (W1.1, W1.2, W1.3; W2.1, W2.2, W2.3; W3.1, W3.2, W3.3) comprises at least one or several of the following work steps:
Creating a cell culture (18), counting the living or dead cells (17), removing cells of the cell culture, detaching the cells from the bottom of a trough of the carrier (1), adding a detaching agent (14), confluence scan (11), adding nutrient (19), discontinuing the cell culture (15) or seeding cells.

9. A system for cultivating biological cell cultures, with a plurality of carriers (1), which each carry one or several cell cultures, with at least one storage module (2), in which the carriers (1) are stored, with a process module (3), in which the cell cultures carried by the carriers (1) are treated by means of at least one treatment device (4), with a transport device (5), with which the carriers (1) are transported between the storage module (2) and the process module (3), and with a control device (6), which has a memory device (7) and which controls the transport device (5) and the treatment device (4), wherein applications (A1, A2, A3) assigned to several different cell cultures are stored in the memory device (7), wherein each application (A1, A2, A3) is a treatment specification for treating the assigned cell culture, which contains one or several workflows (W1.1, W1.2, W1.3; W2.1, W2.2, W2.3; W3.1, W3.2, W3.3), which are performed at time intervals successively at a respective start time (t) in the process module (3), wherein a workflow (W1.1, W1.2, W1.3; W2.1, W2.2, W2.3; W3.1, W3.2, W3.3) contains one work step or several work steps to be performed one after the other at short notice, wherein the control device (6) controls the simultaneous performance of a plurality of applications (A1, A2, A3), and thereby starts one or several workflows (W1.1, W1.2, W1.3; W2.1, W2.2, W2.3; W3.1, W3.2, W3.3) of the different applications (A1, A2, A3) in chronological sequence one after the other in the same process module (3), **characterized in that** each workflow (W1.1, W1.2, W1.3; W2.1, W2.2, W2.3; W3.1, W3.2, W3.3) has assigned to it an active function W(t) that depends on the start time (t), and the sequence and start times (t) of the workflows (W1.1, W1.2, W1.3; W2.1, W2.2, W2.3; W3.1, W3.2, W3.3) of different applications (A1, A2, A3) or instances of the same application are calculated by minimizing or maximizing the sum ($\Sigma$) of active functions (W(t)).

10. The system according to claim 9, **characterized in that** the control device 6 is programmed to implement a method according to one of claims 1 to 8.

11. The system according to claim 9 or 10, **characterized in that** the transport device (5) removes a first carrier (1) for performing a first workflow (W1.1) from a storage space in the storage module (2) at a first time and transports it to the process module (3) in such a way that the treatment device (4) begins to treat the cell culture carried by the first carrier at a first start time (tW1.1), and the transport device (5) then removes a second carrier (1) for performing a second workflow (W2.1) from a storage space in the storage module (2) at a second time and transports it to the process module (3) in such a way that the treatment device (4) begins to treat the cell culture carried by the second carrier (1) at a second start time (t) after the performance of the first workflow (W1.1) has concluded, wherein the two start times (t) are calculated in such a way as to minimize or maximize the sum ($\Sigma$) of the active functions (W(t)).

12. The system according to one of claims 9 to 11, **characterized by** a graphic data input device, with which a user can set the progression of an active function.

13. The system according to one of claims 9 to 12, **characterized in that** the treatment device (4) has a pipetting device, a microscope and/or an opening device for opening nutrient-containing containers, and /or that the storage module (2) is an incubator, and/or that the transport device (5) has a gripping arm or end effector and/or is automated.

14. The system according to one of claims 9 to 13, **characterized in that** the minimum or maximum is a local minimum or maximum, and/or that the minimum or maximum is a value calculated over the duration of a limited number of workflows (W1.1, W1.2, W1.3; W2.1, W2.2, W2.3; W3.1, W3.2, W3.3).

15. Software for a system according to one of claims 9 to 14, which is programmed to implement a method according to one of claims 1 to 8.

# EP 4 308 682 B1

**Revendications**

1. Procédé de mise en culture de cultures cellulaires biologiques, dans lequel plusieurs supports (1) portent chacun une ou plusieurs cultures cellulaires qui sont stockées dans au moins un module de stockage (2), dans lequel les cultures cellulaires d'un module de traitement (3) sont traitées au moyen d'au moins un dispositif de traitement (4), les supports (1) étant transportés avec un dispositif de transport (5) entre le module de stockage (2) et le module de traitement (3), dans lequel, au moyen d'un dispositif de commande (6) comportant un dispositif de mémoire (7), le dispositif de transport (5) et le dispositif de traitement (4) sont commandés et, dans le dispositif de mémoire (7), plusieurs applications (A1, A2, A3) associées à des cultures cellulaires différentes les unes aux autres sont mémorisées, chaque application (A1, A2, A3) étant une instruction de traitement pour le traitement de la culture cellulaire associée, qui comprend un ou plusieurs flux de travail (W1.1, W1.2, W1.3 ; W2.1, W2.2, W2.3 ; W3.1, W3.2, W3.3), qui sont exécutés à intervalles successifs à une heure de début (t) dans le module de traitement (3), un flux de travail (W1.1, W1.2, W1.3 ; W2.1, W2.2, W2.3 ; W3.1, W3.2, W3.3) comprenant une ou plusieurs étapes de travail à exécuter successivement à bref délai, le dispositif de commande (6) commandant l'exécution simultanée d'une pluralité d'applications (A1, A2, A3) et, ce faisant, le ou les flux de travail (W1.1, W1.2, W1.3 ; W2.1, W2.2, W2.3 ; W3.1, W3.2, W3.3) des applications (A1, A2, A3) différentes les unes des autres commençant successivement dans un ordre chronologique dans le même module de traitement (3),
**caractérisé en ce qu'**une fonction opérationnelle (W(t)) dépendant de l'heure de début (t) une est attribuée à chaque flux de travail (W1.1, W1.2, W1.3 ; W2.1, W2.2, W2.3 ; W3.1, W3.2, W3.3) et que la séquence et les heures de début (t) des flux de travail (W1.1, W1.2, W1.3 ; W2.1, W2.2, W2.3 ; W3.1, W3.2, W3.3) de différentes applications (A1, A2, A3) ou d'instances des mêmes applications sont calculées par minimisation ou maximisation de la somme ($\Sigma$) des fonctions opérationnelles (W(t)).

2. Procédé selon la revendication 1, **caractérisé en ce qu'**à chaque flux de travail (W1.1, W1.2, W1.3 ; W2.1, W2.2, W2.3 ; W3.1, W3.2, W3.3) est affectée une heure de début préférée ($t_p$), à laquelle la fonction opérationnelle (W(t) affectée au flux de travail (W1.1, W1.2, W1.3 ; W2.1, W2.2, W2.3 ; W3.1, W3.2, W3.3) présente un minimum ou un maximum.

3. Procédé selon la revendication 2, **caractérisé en ce qu'**au moins certain premiers flux de travail (W1.1, W1.2, W1.3) des différentes applications (A1, A2, A3) présentent une première heure de début préférée ($t_p$) qui tombe dans une période de traitement d'un second flux de travail, dont la seconde heure de début préférée ($t_p$) se situe avant la première heure de début préférée ($t_p$), le dispositif de commande (6) décalant la première et/ou la seconde heure de début de telle sorte que les deux premier et second flux de travail (W1.1, W1.2, W1.3 ; W2.1, W2.2, W2.3 ; W3.1, W3.2, W3.3) sont exécutés successivement, les première et seconde heures de début étant sélectionnés de telle sorte que la somme ($\Sigma$) des fonctions opérationnelles (W(t) soit minimisée ou maximisée.

4. Procédé selon l'une des revendications 2 ou 3, **caractérisé en ce qu'**à au moins certains des flux de travail (W2.1, W2.2, W2.3) est affectée une heure de début la plus précoce ($t_e$), avant laquelle le flux de travail (W2.1, W2.2, W2.3) ne peut être autorisé et/ou une heure de début la plus tardive ($t_i$) est affectée, après laquelle le flux de travail (W2.1, W2.2, W2.3) ne peut être autorisé, étant en particulier prévu que la fonction opérationnelle (W(t)) présente un maximum à l'heure de début la plus précoce ($t_e$) et à l'heure de début la plus tardive ($t_i$).

5. Procédé selon l'une des revendications 2 à 4, **caractérisé en ce que** la fonction opérationnelle (W(t)) présente une branche descendante ou ascendante linéaire ou non linéaire depuis une heure de début la plus précoce ($t_e$) jusqu'à l'heure de début préférée ($t_p$) et une branche ascendante ou montante linéaire ou non linéaire depuis l'heure de début préférée ($t_p$) jusqu'à l'heure de début la plus tardive ($t_i$).

6. Procédé selon l'une quelconque des revendications précédentes, **caractérisé en ce que** le déroulement de la fonction opérationnelle (W(t)) est choisi de telle sorte que les coûts à supporter pour la mise en œuvre de l'application (A1, A2, A3) au début des flux de travail (W1.1, W1.2, W1.3 ; W2.1, W2.2, W2.3 ; W3.1, W3.2, W3.3) soient au minimum aux heures de début préférées ($t_p$) et au maximum à l'heure de début de la plus précoce et la plus tardive.

7. Procédé selon l'une quelconque des revendications précédentes, **caractérisé en ce qu'**au moins certains des flux de travail (W1.1, W2.1, W3.1) comportent une étape de surveillance de la croissance cellulaire dans laquelle la confluence d'une culture cellulaire est déterminée et, en fonction de cela, l'heure de début préférée ($t_p$) d'au moins un flux de travail suivant (W1.1, W2.2, W3.3) est définie et/ou l'heure de début la plus précoce ($t_i$) et/ou l'heure de début la plus tardive ($t_i$) d'un flux de travail suivant (W1.2, W2.2, W3.2) est spécifiée dans l'étape de surveillance.

8. Procédé selon l'une quelconque des revendications précédentes, **caractérisé en ce que** le flux de travail (W1.1, W1.2, W1.3 ; W2.1, W2.2, W2.3 ; W3.1, W3.2, W3.3) comprend au moins une ou plusieurs des étapes de travail suivantes:
création d'une culture cellulaire (18), comptage des cellules vivantes ou mortes (17), prélèvement des cellules de la culture cellulaire, détachement des cellules du fond d'un creux du support (1), ajout d'un agent de démoulage (14), scannage de confluence (11), ajout de nutriments (19), abandon de la culture cellulaire (15) ou semis dans les cellules.

9. Système de culture de cultures cellulaires biologiques, comprenant une pluralité de supports (1) portant chacun une ou plusieurs cultures cellulaires, comportant au moins un module de stockage (2) dans lequel les supports (1) sont stockés, un module de traitement (3) dans lequel les cultures cellulaires supportées par les supports (1) sont traitées au moyen d'au moins un dispositif de traitement (4), avec un dispositif de transport (5) au moyen duquel les supports (1) sont transportés entre le module de stockage (2) et le module de traitement (3) et avec un dispositif de commande (6) comprenant un dispositif de mémoire (7) et commandant le dispositif de transport (5) et le dispositif de traitement (4), plusieurs applications (A1, A2, A3) associées à différentes cultures cellulaires étant enregistrées dans le dispositif de mémoire (7), chaque application (A1, A2, A3) étant une instruction de traitement pour le traitement de la culture cellulaire associée, qui comprend un ou plusieurs flux de travail (W1.1, W1.2, W1.3 ; W2.1, W2.2, W2.3 ; W3.1, W3.2, W3.3) qui sont exécutés respectivement à intervalles successifs à une heure de début (t) dans le module de traitement (3), un flux de travail (W1.1, W1.2, W1.3 ; W2.1, W2.2, W2.3 ; W3.1, W3.2, W3.3) comprenant une ou plusieurs étapes de travail à exécuter successivement dans un bref délai, le dispositif de commande (6) commandant l'exécution simultanée d'une pluralité d'applications (A1, A2, A3), le ou les flux de travail (W1.1, W1.2, W1.3 ; W2.1, W2.2, W2.3 ; W3.1, W3.2, W3.3) des applications différentes les unes des autres (A1, A2, A3) débutant dans un ordre chronologique dans le même module de traitement (3),
**caractérisé en ce qu'**à chaque flux de travail (W1.1, W1.2, W1.3 ; W2.1, W2.2, W2.3 ; W3.1, W3.2, W3.3) est affectée une fonction opérationnelle (W(t)) dépendant de l'heure de début (t) et que la séquence et des heures de début (t) des flux de travail (W1.1, W1.2, W1.3 ; W2.1, W2.2, W3.3 ; W3.1, W3.2, W3.3) de diverses applications (A1, A2, A3) ou d'instances de la même application sont calculées par minimisation ou maximisation de la somme ($\Sigma$) des fonctions opérationnelles (W(t)).

10. Système selon la revendication 9, **caractérisé en ce que** le dispositif de commande (6) est programmé pour la mise en œuvre d'un procédé selon l'une des revendications 1 à 8.

11. Système selon la revendication 9 ou 10, **caractérisé en ce que** le dispositif de transport (5) prélève un premier support (1) pour exécuter un premier flux de travail (W1.1) à un premier moment vers une position de stockage dans le module de stockage (2) et le transporte vers le module de traitement (3) de manière à ce que le dispositif de traitement (4) commence à traiter la culture cellulaire supportée par le premier support à un premier instant de départ (tW1.1) et que le dispositif de transport (5) prélève ensuite un second support (1) pour exécuter un deuxième flux de travail (W2.1) à un deuxième instant un emplacement de stockage dans le module de stockage (2) et le transporte au module de traitement (3) de manière à ce que le dispositif de traitement (4) commence à traiter la culture cellulaire supportée par le second support (1) après la fin de la mise en œuvre du premier flux de travail (W1.1) à une seconde heure de début (t), les deux heures de début (t) étant calculées de telle sorte que la somme ($\Sigma$) des fonctions opérationnelles (W(t)) soit minimisée ou maximisée.

12. Système selon l'une quelconque des revendications 9 à 11, **caractérisé par** un dispositif d'entrée de données graphique, avec lequel un utilisateur peut spécifier le déroulement d'une fonction opérationnelle.

13. Système selon l'une quelconque des revendications 9 à 12, **caractérisé en ce que** le dispositif de traitement (4) présente un dispositif de pipetage, un microscope et/ou un dispositif d'ouverture pour ouvrir des récipients contenant des substances nutritives et/ou **en ce que** le module de stockage (2) est un incubateur et/ou **en ce que** le dispositif de transport (5) présente un bras de préhension ou un effecteur terminal et/ou est automatisé.

14. Système selon l'une quelconque des revendications 9 à 13, **caractérisé en ce que** le minimum ou maximum est un minimum ou maximum local et/ou **en ce que** le minimum ou maximum est une valeur calculée sur la durée d'un nombre limité de flux de travail (W1.1, W1.2, W1.3 ; W2.1, W2.2, W2.3 ; W3.1, W3.2, W3.3).

15. Logiciel pour système selon l'une quelconque des revendications 9 à 14, qui est programmé pour mettre en œuvre un procédé selon l'une quelconque des revendications 1 à 8.

Fig. 1

Fig. 2

# Fig.3

# Fig.4

# Fig.5

# Fig.6

# Fig.7

# Fig. 8

W1  W2  W3  19  W4  11  W5  15

A

10 mins — 24h — 10 mins — 24h — 10 mins — 24h

19  19

1 min

W6  11

if  10-20 mins

5-15 mins

if  if  10-30 mins

16
17
18

14  19  19

11

# Fig. 9

21  22  23

Fig. 10

Fig. 11

**Fig. 12**

A1  W1.1  W1.2  W1.3

A2  W2.1  W2.2  W2.3

A3  W3.1  W3.2  W3.3  a

W1.1  W3.1  W2.1  W2.2  W1.2  W3.2  W2.3  W1.3  W3.3  b

**Fig. 13**

$W(t)$

max

$t_e$  $t_p$  $t_l$  $t$  a

b

0  1  2  3  4  $t$

c

10%  20%  40%  80%  100%  confl.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2005260743 A
- EP 1598415 A1
- WO 2020098960 A1
- WO 2020098957 A1